# EUROPEAN PATENT APPLICATION

(11) **EP 3 594 336 A1**
(43) Date of publication of application: **15.01.2020**
(21) Application number: 18305950.0
(22) Date of filing: 13.07.2018
(51) Int. Cl.: C12N 9/88, C12Q 1/527

(54) **TREATMENT OF DOWN SYNDROME**

(71) Applicant: Université de Strasbourg, 67000 Strasbourg (FR); INSTITUT NATIONAL DE LA SANTE ET DE LA RECHERCHE MEDICALE (INSERM), 75013 Paris (FR); CENTRE NATIONAL DE LA RECHERCHE SCIENTIFIQUE, 75016 Paris (FR); Université de Bretagne Occidentale, 29200 Brest (FR)
(72) Inventor: HÉRAULT, Yann, 67380 LINGOSLHEIM (FR); BLONDEL, Marc, 29200 BREST (FR); FRIOCOURT, Gaëlle, 29200 BREST (FR); MARÉCHAL, Damien, 67400 ILLKIRCH (FR)
(74) Representative: Lavoix

(57) **Abstract**

The present invention relates to eukaryote cell model, method for screening molecules or compositions, molecule counteracting at least one effect and/or consequence of overexpression of Cystathionine beta-synthase (EC 4.2.1.22) and/or of a coding DNA thereof, for use in a treatment of intellectual disability in Down syndrome (DS), pharmaceutical compositions and applications thereof.

## Description

The present invention relates to eukaryote cell model, method for screening molecules or compositions, molecule counteracting at least one effect and/or consequence of overexpression of Cystathionine beta-synthase (EC 4.2.1.22) and/or of a coding DNA thereof, for use in a treatment of intellectual disability in Down syndrome (DS), pharmaceutical compositions and applications thereof.

### Prior art

Located on chromosome 21 in humans, the gene encoding the cystathionine β-synthase (CBS) enzyme is overexpressed in patients with Down syndrome. Two mouse models, trisomic for parts of chromosome 17 murine (on which is located Cbs), present disorders in learning and memory. Overexpression of the Cbs gene alone is capable of disrupting the synaptic transmission and induce memory recognition disorders in mice, suggesting that CBS is a relevant therapeutic target for Down syndrome and that molecules capable of inhibiting CBS activity could be potential drug candidates for improving, at least in part, the cognitive functions of Down syndrome patients. The search for these inhibitors pharmacological approaches has so far been limited to either in vitro enzyme-based or *E*. *Coli*-based approached which allowed only identification of weakly active compounds (IC⁵⁰>100 µM for most of them), contributing probably to explain why no *in vivo* active compound in an animal model has yet been reported. In addition, no screening methods based on eukaryotic cell have yet been described.

Down syndrome (DS), or trisomy 21, is caused by the presence of 3 copies of part or of the entire chromosome 21 and is the most common cause of genetic intellectual disability (approximately 1 in 700 births). In addition to disability, patients have dysmorphia, significant heart, endocrine and immune problems, and a higher risk of developing Alzheimer's disease and leukemias than the general population. Although significant progress has been made in the management of these patients, only one treatment for intellectual disability is currently being studied, with mixed results. To go further with the hope of improving patients' cognitive performance, various studies carried out over the past twenty years, either in humans or on transgenic mice, aim to understand the impact of the overexpression of the various genes of chromosome 21. These studies suggest that the number of genes responsible for intellectual disability in Down syndrome may be limited. One of them, CBS, encodes an enzyme (cystathionine b-synthase) involved in the metabolic pathway of transulfuration leading to cysteine and glutathione synthesis. Indeed, the enzyme CBS catalyzes the condensation of serine and homocysteine to form cystathionine. In mammals, this crucial metabolic pathway allows: i) to avoid the accumulation of homocysteine which is toxic at high concentrations, ii) to regulate methionine homeostasis which is essential for the initiation of translation and also provide the methyl residues necessary for the methylation of DNA and histones and iii) controlling the production of cysteine, which itself is involved in the synthesis of glutathione, the major intracellular antioxidant. Finally, CBS can convert cysteine and homocysteine into cystathionine and H2S, a gas molecule playing an important role in synaptic transmission. CBS is therefore located at a major metabolic junction.

CBS is a 63 kDa protein, consisting of an N-terminal heme binding part, a central part containing the catalytic domain and an auto-inhibiting C-terminal part which, upon binding to AdoMet, loses this inhibition capacity (Shan et al, 2001: Shan X, Dunbrack RL Jr, Christopher SA, Kruger WD (2001). Mutations in the regulatory domain of cystathionine beta synthase can functionally suppress patient-derived mutations in cis. Hum Mol Genet. 10: 635-643.). The functional counterpart of CBS in *S*. *cerevisiae* yeast is encoded by the *CYS4* gene, the inactivation of which leads to an inability of yeast cells to synthesize cysteine. Although human proteins and yeast are not entirely homologous (Cys4 does not have the N-terminal domain of binding to the heme present in the CBS protein), expression of the human CBS gene restores the synthesis of the cysteine in a yeast strain deleted for the *CYS4* gene (Kruger, W. D., and D. R. Cox. 1994. "A yeast system for expression of human cystathionine beta-synthase: structural and functional conservation of the human and yeast genes." Proc Natl Acad Sci USA 91 (14): 6614-8).

Several arguments suggest an important role for CBS in the pathophysiology of Down syndrome. As early as the 1970s, Professor Jérôme Lejeune noted the phenotypic contrast between this syndrome and homocystinuria, an autosomal recessive inherited disease associated with loss of CBS function mutations and characterized by high levels of homocysteine in patients' urine, intellectual impairment and early onset cardiovascular disorders. In addition, some studies have measured lower levels of homocysteine, methionine, S-adenosyl of homocysteine (AdoHCy) and S-adenosylmethionine (AdoMet) while, on the contrary, cystathionine and cysteine levels were higher, consistent with CBS overexpression in these individuals. These observations were also confirmed in a trisomy 21 mouse model (Pereira et al, 2009: Pereira PL, Magnol L, Sahun I, Brault V, Duchon A, Prandini P, Gruart A, Bizot JC, Chadefaux-Vekemans B, Deutsch S, Trovero F, Delgado-Garcia JM, Antonarakis SE, Dierssen M, Herault Y (2009). A new mouse model for the trisomy of the Abcg1-U2af1 region reveals the complexity of the combinatorial genetic code of down syndrome. Hum Mol Genet 18: 4756-4769.). These observations support the hypothesis that the level of expression of this gene is crucial for cognitive functioning.

Since overexpression of CBS contributes to cognitive deficits observed in mouse models of trisomy 21, it appears important to identify drug candidates capable of attenuating its enzymatic activity in vivo.

### Aims of the present invention

The present invention aims to provide a new cellular model to screen drug candidates for the treatment of DS in a human patient.

The present invention aims to provide such new eukaryote models such as yeast model, especially for providing better modelling of DS in mammal and/or human.

The present invention aims to provide CBS inhibitors or drug candidates selected on such models for improving the chances of success for the treatment of DS in a human patient.

Also the present invention aims to provide a better understanding of DS and model thereof notably to provide better drug candidates for the treatment of DS in a human patient.

### Description of the invention

The present invention relates to a non human eukaryote cell model, said eukaryote cell overexpressing Cystathionine beta-synthase (EC 4.2.1.22) and/or a coding DNA thereof.

In one embodiment, the Cystathionine Beta Synthase coding DNA is a human Cystathionine Beta Synthase gene (Cbs) coding DNA (NC_001139.9, Ensembl: ENSG00000160200)

In one embodiment, the Cystathionine Beta Synthase is human Cystathionine Beta Synthase protein (CBS) (UniProtKB: P35520) (SEQ ID N0:1).

In one embodiment, a particular homologue of human CBS is yeast cystathionine beta-synthase protein (*CYS4* ; coded by X72922.1 S.cerevisiae gene for cystathionine beta-synthase) (SEQ ID N0:2). Yeast Cystathionine Beta Synthase protein has a similar function as the human protein (Kruger, W. D., and D. R. Cox. 1994. "A yeast system for expression of human cystathionine beta-synthase: structural and functional conservation of the human and yeast genes." Proc Natl Acad Sci U S A 91 (14): 6614-8).

The expression "functional eukarotic gene or protein equivalent thereof" means notably a functional gene or protein equivalent to human Cbs gene or CBS protein found in an eukaryote, and in particular a gene expressing a protein catalyzing synthesis of cystathionine from serine and homocysteine in said eukaryote.

In one embodiment, "functional eukaryotic gene or protein equivalent thereof" means in particular at least that gene or protein is homologue to human Cbs gene or CBS protein.

"Homologue" or "homologous" or "homology" or "identity" or "similarity" refers to sequence similarity between two peptides or between two nucleic acid molecules. Homology can be determined by comparing a position in each sequence which may be aligned for purposes of comparison. When a position in the compared sequence is occupied by the same base or amino acid, then the molecules are homologous at that position. A degree of homology between sequences is a function of the number of matching or homologous positions shared by the sequences. An "unrelated" or "non-homologous" sequence shares less than 40% identity, or alternatively less than 25% identity, with one of the sequences of the present invention.

A polynucleotide or polynucleotide region (or a polypeptide or polypeptide region) has a certain percentage (for example, 70%, 75%, 80%, 85%, 90%, 95%, 98% or 99%) of "sequence identity" to another sequence means that, when aligned, that percentage of bases (or amino acids) are the same in comparing the two sequences. This alignment and the percent homology or sequence identity can be determined using software programs known in the art, for example those described in Ausubel et al. eds. (2007) Current Protocols in Molecular Biology. Default parameters can be used for alignment. One alignment program is BLAST, using default parameters. Exemplary programs include, but are not limited to, BLASTN and BLASTP. Details of these programs can be found at the following Internet address: https://www.ncbi.nlm.nih.gov/ or https://blast.ncbi.nlm.nih.gov/Blast.cgi. An equivalent nucleic acid, polynucleotide or oligonucleotide is one having at least 80% sequence identity, or alternatively at least 85% sequence identity, or alternatively at least 90% sequence identity, or alternatively at least 92% sequence identity, or alternatively at least 95% sequence identity, or alternatively at least 97% sequence identity, or alternatively at least 98% sequence identity to the reference nucleic acid, polynucleotide, or oligonucleotide.

A "gene" refers to a polynucleotide containing at least one open reading frame (ORF) that is capable of encoding a particular polypeptide or protein after being transcribed and translated.

The term "express" refers to the production of a gene product.

As used herein, "expression" refers to the process by which polynucleotides are transcribed into mRNA and/or the process by which the transcribed mRNA is subsequently being translated into peptides, polypeptides, or proteins. If the polynucleotide is derived from genomic DNA, expression may include splicing of the mRNA in a eukaryotic cell.

"Complementarity" or "homology" (the degree that one polynucleotide is complementary with another) is quantifiable in terms of the proportion of bases in opposing strands that are expected to form hydrogen bonding with each other, according to generally accepted base-pairing rules.

Yeast cystathionine β-synthase *(CYS4)* coding sequence was amplified by PCR from the genomic DNA (NC_001139.9) of the W303 strain (genetic background: *MATa, leu2-3,112 trp1-1 can1-100 ura3-1 ade2-1 his3-11,15*) using the following primers: Bam-Cys4-F: CGGGATCCCGATGACTAAATCTGAGCAGCAAG (SEQ ID NO:3) and Xho-Cys4-R: GCCTCGAGTCTTATGCTAAGTAGCTCAGTAAATCC (SEQ ID NO:4), which introduced *Bam*HI and *Xho1* restriction sites. *CYS4* coding sequence was then subcloned under the control of the strong constitutive *GDP* promoter (Mumberg, D., R. Muller, and M. Funk. 1995. "YEAST VECTORS FOR THE CONTROLLED EXPRESSION OF HETEROLOGOUS PROTEINS IN DIFFERENT GENETIC BACKGROUNDS." Gene 156 (1):119-122) in yeast expression plasmids pRS424 (TRP1) and pRS426 (*URA3*) (Sikorski RS, Hieter P. A system of shuttle vectors and yeast host strains designed for efficient manipulation of DNA in Saccharomyces cerevisiae. Genetics. (1989) 122:19-2798; Christianson TW, Sikorski RS, Dante M, Shero JH, Hieter P. Multifunctional yeast high-copy-number shuttle vectors. Gene. (1992) 110:119-122) which are both 2 micron (2 µ) high copy number vectors, that is to say that they are present at ∼50 copies per cell.

In one embodiment, yeast cells are transformed with plasmids. Accordingly, the term "vector" refers in particular to plasmids according to the present invention.

More specifically, for example, yeast cells of the W303 genetic background were transformed with these two plasmids using a standard lithium acetate method (Gietz D, St Jean A, Woods RA, Schiestl RH. Improved method for high efficiency transformation of intact yeast cells. Nucleic Acids Res. (1992) 20:1425). Transformed cells were then selected on solid medium lacking uracil and tryptophan (Synthetic Dextrose Minimal medium (SD medium) composed of 0.67% (w/v) *Yeast* Nitrogen Base w/o amino acids and complemented with 0.1% (w/v) casamino acid, 40 mg/l adenine and 2% (v/v) glucose).

In the invention, it was observed that the overexpression of *CYS4* leads to a decreased intracellular level of methionine, similarly to what has been shown in DS patients. Therefore yeast cells overexpressing Cys4 become methionine auxotroph and thereby are unable to grow on methionine-free minimal media.

In one embodiment, said Cystathionine beta-synthase coding gene is Cystathionine b-synthase gene *(CYS4)* (Systematic Name *YGR155W* ; SGD ID SGD:S000003387) or an homologue gene overexpressing said Cystathionine beta-synthase.

In one embodiment, said model comprises or consists of *CYS4*-overexpressing cells.

In one embodiment, said Cystathionine b-synthase gene *(CYS4)* is from yeast, in particular *Saccharomyces cerevisiae.*

In one embodiment, said eukaryote cell is a yeast, in particular *Saccharomyces cerevisiae.*
In one embodiment, *CYS4* is expressed from the (strong) constitutive *GPD* promoter. For example, this promoter is the one of the *TDH3* gene encoding Glyceraldehyde-3-phosphate dehydrogenase, isozyme 3 protein. This promoter enables, in our model, a strong constitutive (that is to say, which does not need to be induced) expression of *CYS4.*

In one embodiment, said yeast cells were grown in uracil- and tryptophan-free minimal liquid medium (SD-Ura/Trp).

Preferably, p424-GPD and p426-GPD 2 µ plasmids are used for preparing said eukaryote cell.

In one embodiment, yeast cells overexpressing *CYS4* are spread on a solid agar-based methionine-free minimal medium.

In one embodiment, sterile filters (Thermo Fisher similar to those used for antibiograms) are put on the agar surface and different drugs from chemical libraries are added on each filters.

Preferably, after adding potentially active compounds to be screened, the yeast are incubated, for example at 33°C, and active compounds are identified by a halo of restored/enhanced growth around the filter on which they are loaded.

The present invention also relates to a method for screening molecules or compositions, said method comprising screening said molecules or compositions with an eukaryote cell model as defined in the present invention.

In one embodiment, said method is for screening molecules or compositions for the treatment of intellectual disability in Down syndrome (DS).

Typically said method comprises a Western Blot analysis, in particular including detection of Cbs (gene)and/or CBS (protein) and/or *CYS4* (gene) and/or Cys4 (protein).

Preferably, said method comprises detecting at least one effect and/or consequence of Cbs and/or *CYS4* overexpression.

For example, an effect and/or consequence of Cbs and/or *CYS4* overexpression is determined by a deficit by novel object recognition memory and/or decreased locomotor activity notably during dark, for example as assesed in model mice overexpressing CBS.

In one embodiment, the screening medium is supplemented with a Cys4p/CBS substrate, preferably with serine.

As an example, for the drug screening, yeast cells transformed with p424-GPD and p426-GPD plasmids containing yeast CBS *(CYS4)* coding sequence were grown in uracil- and tryptophan-free minimal liquid medium (SD-Ura/Trp) overnight at 29°C. The following day, cells were diluted to OD₆₀₀∼0.2 in fresh medium and grown for 4 hours to reach exponential phase. Then three hundred and fifty microliters of exponentially growing yeast cells overexpressing *CYS4,* adjusted to an OD₆₀₀ of 0.5, were spread homogeneously with sterile glass beads (a mix of ∼1.5 and 3 mm diameter) on a square Petri dish (12 cm × 12cm) containing uracil-, tryptophan- and methionine-free minimal agar-based solid medium (SD-UrarTrp/Met). In addition, to get a degree of methionine auxotrophy sufficient to allow an efficient screening, the screening medium was supplemented with serine (2% (w/v)), which is one of CYS4/CBS substrates. Sterile filters (Thermo Fisher similar to those used for antibiograms) were placed on the agar surface, and 2 µl of individual compound from the various chemical libraries were applied to each filter. On each Petri plate, DMSO, the vehicle, was added as a negative control on the top left filter, and 2 nmol of methionine as a positive control on the bottom right filter. After 3 days of incubation at 33°c, active compounds are identified by a halo of restored/enhanced growth around the filter on which they were loaded.

In one embodiment, said eukaryote cell has a degree of methionine auxotrophy sufficient to allow an efficient screening.

In one embodiment, said method comprising detecting molecules or compositions suppressing a methionine auxotrophy induced by *CYS4* overexpression.

In one embodiment, the growth medium for screening molecules on the eukaryote cell model contains Cys4p/CBS substrates, which is for example serine.

Preferably, said growth medium is a solid agar-based methionine-free minimal medium.

For example, said growth medium contains uracil-, tryptophan- and methionine-free minimal agar-based solid medium (SD-Ura/Trp/Met) containing 2% (w/v) serine.

The present invention also relates to a molecule (inhibitor) inhibiting CBS, and more particularly counteracting at least one effect and/or consequence on the phenotype of a patient presenting a *CYS4*/CBS overexpression. The present invention also relates to a molecule counteracting at least one effect and/or consequence on the phenotype of a patient presenting Down syndrome.

The present invention also relates to a molecule counteracting an intellectual disability in a patient presenting Down syndrome.

It should be understood by "inhibiting CBS" or "CBS inhibitor" that the active molecule may not directly inhibit CBS enzymatic activity but rather acts on the cellular consequences of CBS overexpression, and more particularly on the consequence on the phenotype of CBS overexpression. In one embodiment, the CBS inhibitor according to the invention is thus a molecule (or drug) acting both directly or not on CBS/Cys4.

The present invention also relates to a molecule counteracting at least one effect and/or consequence of overexpression of Cystathionine beta-synthase (EC 4.2.1.22) and/or of a coding DNA thereof, for use in a treatment of intellectual disability in Down syndrome (DS).

In one embodiment, said Cystathionine beta-synthase is human Cystathionine Beta Synthase protein (CBS) and/or a functional eukarotic protein equivalent thereof, for use in a treatment of intellectual disability in Down syndrome (DS).

In one embodiment, said DNA coding for Cystathionine beta-synthase is human Cystathionine Beta Synthase gene (Cbs) and/or a functional eukarotic gene equivalent thereof, for use in a treatment of intellectual disability in Down syndrome (DS).

In one embodiment, said molecule suppresses a methionine auxotrophy induced by Cys4p overexpression in an eukaryote cell model as defined in the present invention, for example according to a method as defined in the present invention.

In one embodiment, counteracting at least one effect and/or consequence of overexpression of Cystathionine beta-synthase (EC 4.2.1.22) and/or of a coding DNA thereof is not achieved by limiting overexpression of Cystathionine beta-synthase (EC 4.2.1.22) and/or of a coding DNA thereof.

In one embodiment, counteracting at least one effect and/or consequence of overexpression of Cystathionine beta-synthase (EC 4.2.1.22) and/or of a coding DNA thereof is achieved by interfering with the phenotypical consequence of overexpression of Cystathionine Beta Synthase gene (Cbs) and/or of an homologue (especially yeast *CYS4* gene) thereof in an eukarotic cell.

In one embodiment, a molecule according to the invention is for use for inhibiting CBS, in particular for treating a DS phenotype, especially in a human subject.

In one embodiment, a molecule according to the invention is for use for treating a DS phenotype, especially in a human subject.

In one embodiment, a molecule according to the invention is for use for treating phenotypical consequences of CBS overexpression, especially in a human subject.

In one embodiment, a molecule according to the invention enables to limit the deficit in object recognition for example as assessed in model mice overexpressing CBS by novel object recognition memory and/or decreased locomotor activity notably during dark phase.

The present invention also relates to a pharmaceutical composition comprising one or more molecules as defined in the present invention, for use in a treatment of intellectual disability in Down syndrome (DS).

The present invention also relates to a pharmaceutical composition comprising one or more molecules as defined in the present invention, said composition optionally further comprising a pharmaceutically active molecule limiting intellectual disability in DS, and for example a molecule inhibiting D*yrk1a* gene and/or DYRK1A protein.

The present invention also relates to a method for the therapeutic treatment of intellectual disability in Down syndrome (DS), said method comprising administering to human or animal body, in particular a mammal, an effective amount of one or more molecules of the invention. Such method includes any embodiments and/or preferred features described with respect to the molecules or compositions according to the present invention, without limitation, including any combination thereof.

The present invention also relates to a method for preparing a pharmaceutical composition for use in a method for the therapeutic treatment as defined in the present invention, said method comprising preparing a pharmaceutical composition comprising one or more molecules as defined in the present invention.

Typically, a pharmaceutical composition comprises pharmaceutically acceptable carrier (also know as excipients).

As used herein, the term "pharmaceutically acceptable carrier" encompasses any of the standard pharmaceutical carriers, such as a phosphate buffered saline solution, water, and emulsions, such as an oil/water or water/oil emulsion, and various types of wetting agents. The compositions also can include stabilizers and preservatives. For examples of carriers, stabilizers and adjuvants, see Remington's Pharmaceutical Sciences (20th ed., Mack Publishing Co. 2000).

A "subject," "individual" or "patient" is used interchangeably herein, and refers to a vertebrate, such as a mammal. Mammals include, but are not limited to, murines, rats, rabbit, simians, bovines, ovine, porcine, canines, feline, farm animals, sport animals, pets, equine, primates, and humans. In embodiments, the mammals include horses, dogs, and cats. In another embodiment of the present invention, the mammal is a human patient.

"Administering" is defined herein as a means of providing an agent or a composition containing the agent to a subject in a manner that results in the agent being inside the subject's body. Such an administration can be by any route including, without limitation, oral, transdermal (e.g., vagina, rectum, oral mucosa), by injection (e.g., subcutaneous, intravenous, parenterally, intraperitoneally, into the CNS), or by inhalation (e.g., oral or nasal). Pharmaceutical preparations are, of course, given by forms suitable for each administration route.

"Treating" or "treatment" of a disease includes: (1) preventing the disease, i.e., causing the clinical symptoms of the disease not to develop in a patient that may be predisposed to the disease but does not yet experience or display symptoms of the disease; (2) inhibiting the disease, i.e., arresting or reducing the development of the disease or its clinical symptoms; or (3) relieving the disease, i.e., causing regression of the disease or its clinical symptoms.

An "effective amount" or "therapeutically effective amount" is an amount sufficient to effect beneficial or desired results. An effective amount can be administered in one or more administrations, applications or dosages. Such delivery is dependent on a number of variables including the time period for which the individual dosage unit is to be used, the bioavailability of the therapeutic agent, the route of administration, etc. It is understood, however, that specific dose levels of the therapeutic agents of the present invention for any particular subject depend upon a variety of factors including the activity of the specific compound employed, the age, body weight, general health, sex, and diet of the subject, the time of administration, the rate of excretion, the drug combination, and the severity of the particular disorder being treated and form of administration. Treatment dosages generally may be titrated to optimize safety and efficacy. Typically, dosage-effect relationships from in vitro and/or in vivo tests initially can provide useful guidance on the proper doses for patient administration. In general, one will desire to administer an amount of the compound that is effective to achieve a serum level commensurate with the concentrations found to be effective in vitro. Determination of these parameters is within the skill of the art.

### Examples

### MATERIALS AND METHODS

For all these tests, mice were kept in Specific Pathogen free conditions with free access to food and water. The light cycle was controlled as 12 h light and 12 h dark (lights on at 7AM). All the behavioural tests were done between 9:00 AM and 4:00 PM.

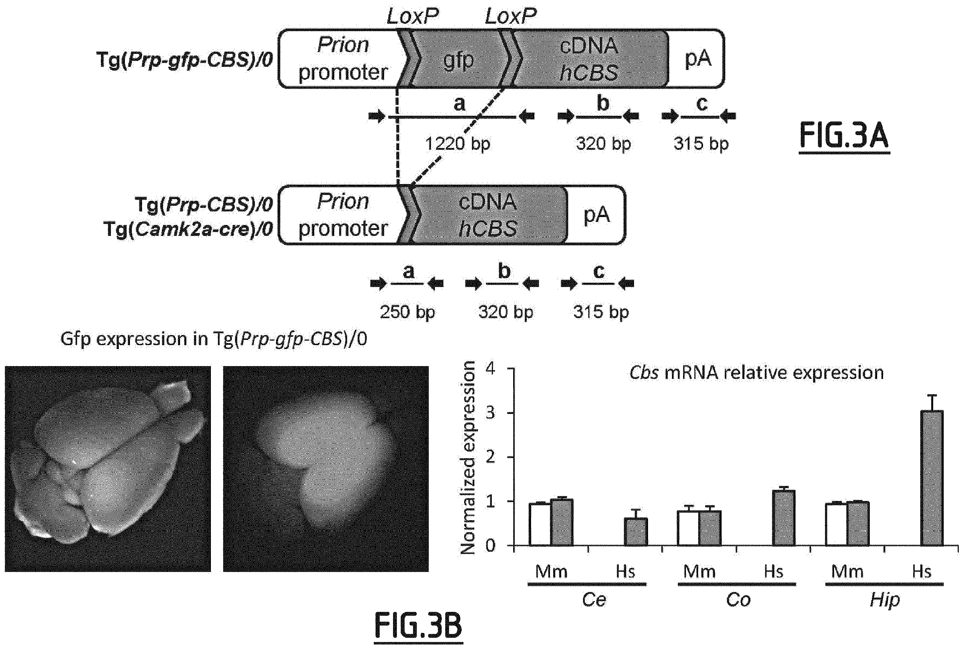

Several mouse lines were used to decipher the influence of *Cbs:* the trisomic mouse model, Dp(17Abcg1-Cbs)1Yah, named here Dp1Yah, carries a segmental duplication of the *Abcg1-Cbs* region of the Mmu17 (Pereira et al. 2009) kept on the C57BU6J; the inactivated allele of C57BL/6J.*Cbs^{tm1Unc}* (Watanabe et al. 1995); and the PAC transgenic line Tg(*CBS*)11181 Eri (named here Tg(CBS)), originally identified as 60.4P102D1 (Butler et al. 2006) and backcrossed on C57BL/6J for more than 7 generations. the transgenic mouse line Tg(*Prp-gfp-CBS*)95-157ICS, named here Tg(*Prp-gfp-CBS*), was designed, generated and selected to overexpress the human *CBS* cDNA from the murine prion promoter region (containing a 8477 bp region upstream of the ATG of the murine prion gene, ie 6170 bp promoter region, exon1, intron 1 and beginning of exon 2) after the excision of a IoxP-gfp-*IoxP* interrupting cassette (Figure 3A) on C57BL/6J background. The transgenic Tg(Camk2a-cre)4Gsc mouse line (Mantamadiotis et al. 2002), named here *Tg(Camk2a-cre),* was used and bred further on C57BL/6J, as a glutamatergic neuron-specific Cre driver. The Dyrk1a BAC transgenic mouse line, named here *Tg*(*Dyrk1a*) was generated previously in present inventor's lab (Guedj et al. 2012). All lines were generated and bred on the C57BL/6J genetic. The genotype identification was done from genomic DNA isolated from tail biopsies with specific PCR reaction (Table 1).

Cbs sequence in the transgenic mice was Human Cbs coding for HUMAN CBS Uniprot P35520 with 551aa).

### Behavioural analysis

To investigate the role of *Cbs* in the Dp1 Yah cognitive phenotypes, 2 independent cohorts were generated (cohort 1 (C1): wild type (wt) littermates n=11; *Cbs*^{*tm1Unc*/+}*,* n= 8; Dp1Yah, n=8; Dp1*Yah*/*Cbs^{tm1Unc},* n=11; and cohort 2 (C2): wt littermates n=18; *Cbs*^{*tm1Unc*/+}*,* n=15; Dp1Yah, n=15; Dp1Yah/*Cbs^{tm1Unc},* n=10). All cohorts were evaluated in the open field (C1: 33 weeks; C2: 14-16 weeks), Novel Object Recognition (NOR) (C1: 33 weeks; C2:14-16 weeks) in adult mice. In addition the Y maze (C2: 15-19 weeks) and the rotarod tests (C2: 25-28 weeks of age) were performed.

Wild-type littermates (n=13) and Tg(CBS)/0 (n=17) hemizygotes were tested for circadian actimetry (14 weeks), Y Maze (16 weeks), open field (17 weeks) and NOR (17 weeks). An additional group of wt (n=9) and Tg(CBS)/0 (n=10) was added to validate the results from the NOR; animals were tested at the same age (17 weeks). A cohort with 4 genotypes (wt (n=13), Tg(Camk2-Cre)/0 (n=11), Tg(*Prp-gfp-CBS*)/0 (n=12), and Tg(Camk2-Cre)/0;*Tg*(Prp*-gfp-CBS*)/0 (n=14)) was evaluated through the same behavioural tests with rotarod (14 weeks), Y maze (16 weeks), open field (19-20 weeks) and NOR (19-20 weeks). 14 wt, 15 *Tg(Dyrkla),* 13 Dp1Yah and 13 Dp1Yah/Tg(*Dyrk1a*) mutant mice were evaluated for open field exploration (11-12 weeks), novel object recognition (11-12 weeks) and Y maze (13 weeks). A second independent cohort with 11 wt, 10 Tg(*Dyrk1a*), 14 Dp1Yah and 10 Dp1Yah/Tg(*Dyrk1a*) was used for Morris water maze learning (14-16 weeks). The behavioural protocols for open-field, Y maze and novel object recognition, rotarod, water maze were are detailed in the supplementary data or elsewhere (Marechal et al. 2015, Duchon et al. 2011).

### Drug screening in yeast

All plasmids were generated using standard procedures. Restriction enzymes and Taq polymerase were obtained from New England Biolabs (Evry, France). T4 DNA ligase was purchased from Promega and purified synthetic oligonucleotides from Eurogentec. Routine plasmid maintenance was carried out in DH5α and TOP10 bacteria strains. Yeast cystathionine b-synthase (*CYS4*) coding sequence was amplified from the genomic DNA of the W303 *WT* strain (see genotype below) using Bam-Cys4-F: CGGGATCCCGATGACTAAATCTGAGCAGCAAG (SEQ ID NO:3) and Xho-Cys4-R: GCCTCGAGTCTTATGCTAAGTAGCTCAGTAAATCC (SEQ ID NO:4) (that introduced *Bam*HI and *Xho1* restriction sites) and subcloned in the high copy number 2 µ-derived vectors p424-GPD and p426-GPD, each time under the control of the strong constitutive *GDP* promoter (Mumberg, Muller, and Funk 1995). Transformation of yeast cells was performed using a standard lithium acetate method (Ito et al. 1983).

The yeast strain used in this study is derived from the W303 *WT* strain: *MATa, leu2-3,112 trp1-1 can1-100 ura3-1 ade2-1 his3-11,15.* The media used for yeast growth were: YPD [1% (w/v) yeast extract, 2% (w/v) peptone, 2% (w/v) glucose], for untransformed cells and Synthetic Dextrose *Minimal medium* (SD medium) [0.67% (w/v) *Yeast* Nitrogen Base w/o amino acids and complemented with 0.1% (w/v) casamino acid, 40 mg/l adenine and 2% (v/v) glucose] for *CYS4*-transformed cells. Solid media contained in addition 2% (w/v) agar.

For the drug screening, yeast cells were grown in uracil- and tryptophan-free minimal liquid medium (SD-Ura/Trp) in overnight liquid cultures at 29 °C. The following day, cells were diluted to OD₆₀₀∼0.2 in in fresh medium and grown for 4 hours to reach exponential phase. Then three hundred and fifty microliters of exponentially growing yeast cells overexpressing Cys4, adjusted to an OD₆₀₀ of 0.5, were spread homogeneously with sterile glass beads (a mix of ∼1.5 and 3 mm diameter) on a square Petri dish (12 cm × 12cm) containing uracil-, tryptophan- and methionine-free minimal agar-based solid medium (SD-UraATrp/Met) containing 2% (w/v) serine. Sterile filters (Thermo Fisher similar to those used for antibiograms) were placed on the agar surface, and 2 µl of individual compounds from the various chemical libraries were applied to each filter. In addition, for each Petri plate, DMSO, the vehicle, was added as a negative control on the top left filter, and 2 nmol of methionine as a positive control on the bottom right filter. Plates were then incubated at 33 °C for 3 days and scanned using a Snap Scan1212 (Agfa).

Two repurposed drug libraries were screened: the Prestwick Chemical Library® (1200 drugs) and the BIOMOL's FDA Approved Drug Library (Enzo Life Sciences, 640 drugs). In addition, the Prestwick Phytochemical library (320 natural "green" compounds, most of them being in use in Human) was also screened. The compounds were supplied in 96-well plates as 10 mM (for the two Prestwick® libraries) and 2 mg/ml (BIOLMOL®) DMSO solutions. Disulfiram was purchased from Sigma-Aldrich and resuspended in DMSO.

### Mouse model treatment with Disulfiram (DSF)

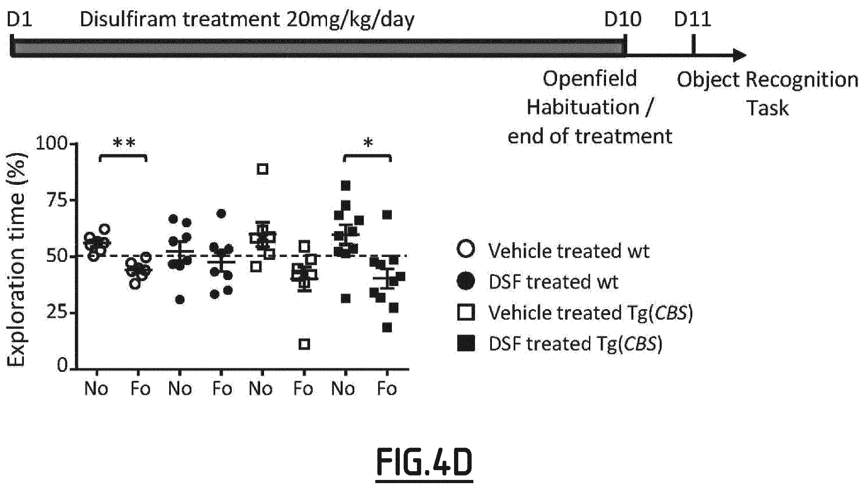

A pre-clinical protocol was designed to target cognitive defects correlated to CBS overexpression in Tg(*CBS*) mice brain (figure 4D).The selected molecule was Disulfiram (DSF), a potent inhibitor of mitochondrial aldehyde dehydrogenase (ALDH) used for the treatment of chronic alcoholism. The invention experiment were in part based on the work of Kim et al. (2010) in which the DSF effect on ethanol sensitization in mice was demonstrated.

Behavioural studies were conducted in 12-16 week old animals; to do so, 3 independent cohorts were generated, in which 4 conditions were tested taking into account the dose of DSF (or vehicle alone) and the genotype. For the cohorts (C1 to C3), respectively 5,7,3 (n=15 in total) wild type (wt) treated with vehicle, 5,3,6 (n=14 in total) transgenic for human *CBS* (*Tg*(*CBS*)) treated with vehicle, 7,5,3 (n=15 in total) wt treated with 10mg/kg/day of DSF, 6,6,8 (n=20 in total) Tg(*CBS*) treated with 10mg/kg/day of DSF based on the dose previously administrated in the reference publication (Kim and Souza-Formigoni 2010) were. Produced. All assessments were scored blind to genotype and treatment as recommended by the ARRIVE guidelines (Karp et al. 2015, Kilkenny et al. 2010). DSF was prepared at 10 mg/mL in DMSO, aliquoted and stored below -20°C. The final formulation was prepared just prior to use as a 1 mg/mL solution diluted in Cremophor EL Castor oil (BASF)/H2O ready for injection (15/75), to reach a final DMSO/Cremophor/H2O 10/15/75 (v/v/v) mix. Treated animals received a daily dose (10 days) of this formulation by intra-peritoneal injection of 10 mg/kg/day. Non-treated animals received the same formulation without DSF. On day 10 of treatment, the animal were habituated 30 min into the arena. On day 11, animals were tested in NOR paradigm to assess recognition memory after 1hour retention as described in the Openfield and Object recognition task protocols (Supplementary data).

### Example 1 - Inducing cognitive impairments

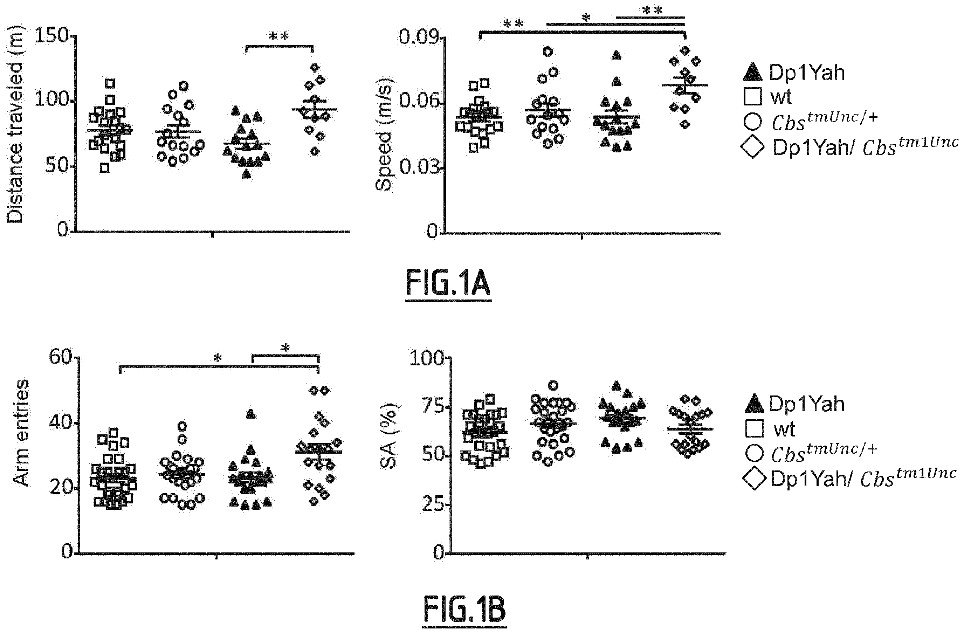

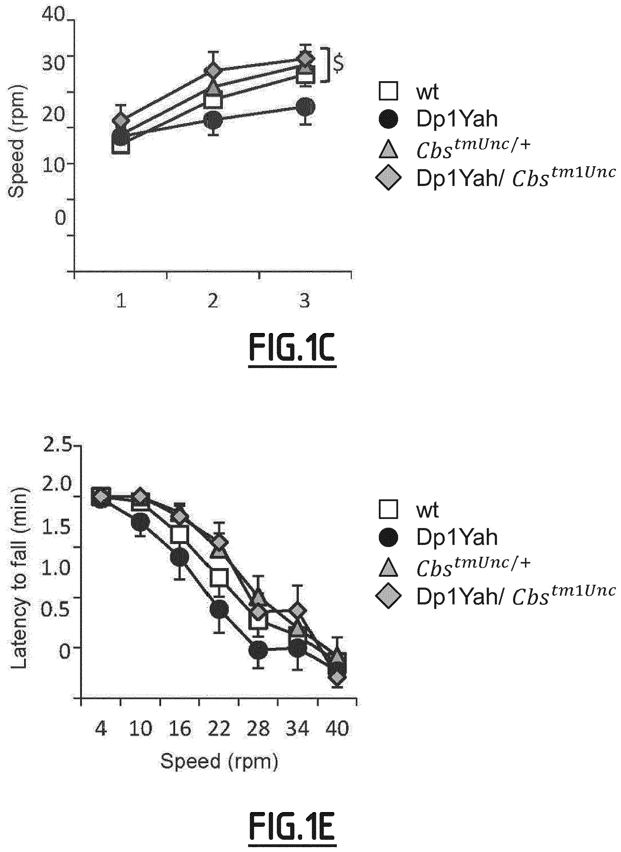

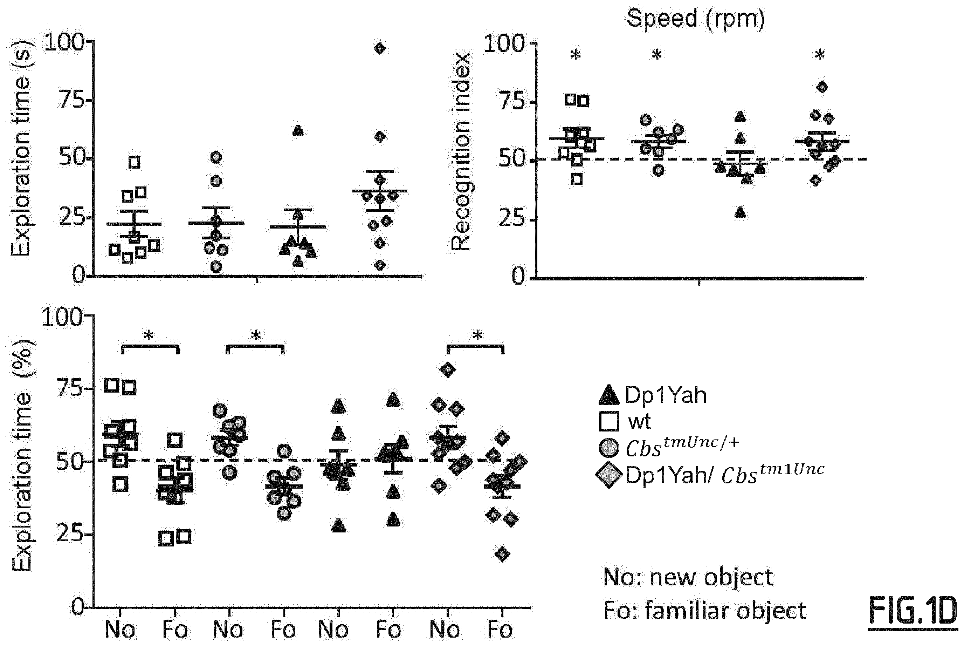

In order to challenge the hypothesis that three copies of *Cbs* are necessary to induce behavioural deficits in the Dp1Yah mice, the Dp1Yah mice were combined with the *Cbs*^{*tm1Unc*/+} knock-out model (Watanabe, M., J. Osada, Y. Aratani, K. Kluckman, R. Reddick, M. R. Malinow, and N. Maeda. 1995. "MICE DEFICIENT IN CYSTATHIONINE BETA-SYNTHASE - ANIMAL-MODELS FOR MILD AND SEVERE HOMOCYST(E)INEMIA." Proceedings of the National Academy of Sciences of the United States of America 92 (5):1585-1589. doi: 10.1073/pnas.92.5.1585. and the Dp1Yah with Dp1Yah/*Cbs^{tm1Unc}* (in which only two copy of *Cbs* are functional), wild type (wt) and *Cbs*^{*tm1Unc*/+} heterozygote controls were compared. In the open field test, most of the genotypes displayed similar exploratory behaviour, except for the Dp1Yah/*Cbs* mice that travelled more distance in the open field arena with a higher speed (Figure 1A left panel; On way ANOVA on distance, post hoc Tukey Test: Dp1Yah vs Dp1Yah/*Cbs*^{+/*tm1Unc*} p=0.002; Figure 1A right panel; On way ANOVA on speed, post hoc Tukey Test: wt vs Dp1Yah/*Cbs*^{+/*tm1Unc*} p=0.004; *Cbs*^{*+*/*tm1Unc*} vs Dp1Yah/*Cbs*^{+/*tm1Unc*} p=0.05; Dp1Yah vs Dp1Yah/*Cbs*^{+/*tm1Unc*} p=0.007). Similarly when the mice performed the Y maze, the increased activity was confirmed with a higher number of arm entries for the Dp1Yah/*Cbs^{tm1Unc}* compared to the other genotypes (Figure 1B; Kruskal-Wallis One way ANOVA on Ranks - genotypes, post hoc Dunn's method: wt vs Dp1Yah/*Cbs*^{+/*tm1Unc*} p<0.05; Dp1Yah vs Dp1Yah/*Cbs*^{+/*tm1Unc*} p<0.05) but no impact on spontaneous alternation (One way ANOVA, F(3,87)=2.486 p=0.066). To determine if motor activity was altered in the Dp1Yah/*Cbs^{tm1Unc}* model, the rotarod test was used. After the first day of training we did not find any change in the maximum speed reached before falling for all tested genotypes (Figure 1C; Speed: repeated measures ANOVA variable « genotype » and « day », F(3;110)=1.816 p=0.155). Nevertheless, a decrease was observed in the locomotor learning in the Dp1Yah mice comparing to the next following days of training which was rescued in the Dp1Yah/*Cbs^{tm1Unc}* mutant (Figure 1C; Speed : repeated measures 2 way ANOVA variable « genotype » and « day », F(2;165)=17.171 p<0.001 post hoc Tuckey method wt «day1 vs day3» p=0.002; *Cbs*^{*tm1Unc*/+} «day1 vs day3» p<0,001; Dp1Yah «day1 vs day3» p=0.238; Dp1Yah/*Cbs^{tm1Unc}* «day1 vs day3» p=0.017). During the test phase, it was found that the Dp1Yah individuals showed a weaker performance compared to *Cbs*^{*tm1Unc*/+} and Dp1Yah/*Cbs^{tm1Unc}* (ANOVA, variable « speed » and « genotype » F(3;385)=5.544 p<0.001 post hoc Tuckey method; «wt vs Dp1Yah» p=0.099; « *Cbs*^{*tm1Unc*/+} vs Dp1Yah » p=0.001; «Dp1Yah vs Dp1Yah/*Cbs^{tm1Unc}*» p=0.01). Then the object memory was tested. No difference was observed during the exploration of the familiar object in the presentation phase of the test (Figure 1D top left panel). However, during the discrimination phase, after 1h of retention, the Dp1Yah mutant mice were not able to differentiate the familiar versus the novel object whereas the wt, *Cbs*^{*tm1Unc*/+} and the Dp1Yah/*Cbs^{tm1Unc}* spent significantly more time on the new object compared to the familiar one (Figure 1D, left bottom panel; two ways ANOVA, variables "genotype" and "objects": F(3;56)= 2.86 with p=0.045; post hoc Tuckey method wt "fam vs new" q= 4.885 and p= 0.001; *Cbs*^{*tm1Unc*/+} q= 3.913 and p= 0.008; Dp1Yah, q= 0,503 and p= 0.724; Dp1Yah/*Cbs*^{*tm1Unc*/+} q= 4.715 and p= 0.002). Accordingly, the recognition index showed that the restoration of two functional copies of *Cbs* in the Dp1Yah mice rescued memory performance in object recognition (Figure 1D right panel; One sample t-test: wt p=0.05; *Cbs*^{*tm1Unc*/+} p= 0.01; Dp1Yah p=0.82; Dp1Yah/*Cbs*^{*tm1Unc*/+} p=0.05).

Overall this set of experiments demonstrated that 3 copies of *Cbs* were necessary for inducing the Dp1Yah phenotypes in novel object recognition. In addition rescuing *Cbs* dosage induced a slight hyperactive phenotype during the exploration of a new environment and restored performance in the rotarod activity. Interestingly, returning back to wt level of expression of *Cbs* in the *Abcg1-Cbs* region enables another trisomic gene from this region to impact on the exploratory behaviour of the mouse.

### Example 2 - The sole overexpression of a human CBS transgene impacts the object recognition and the locomotor activity.

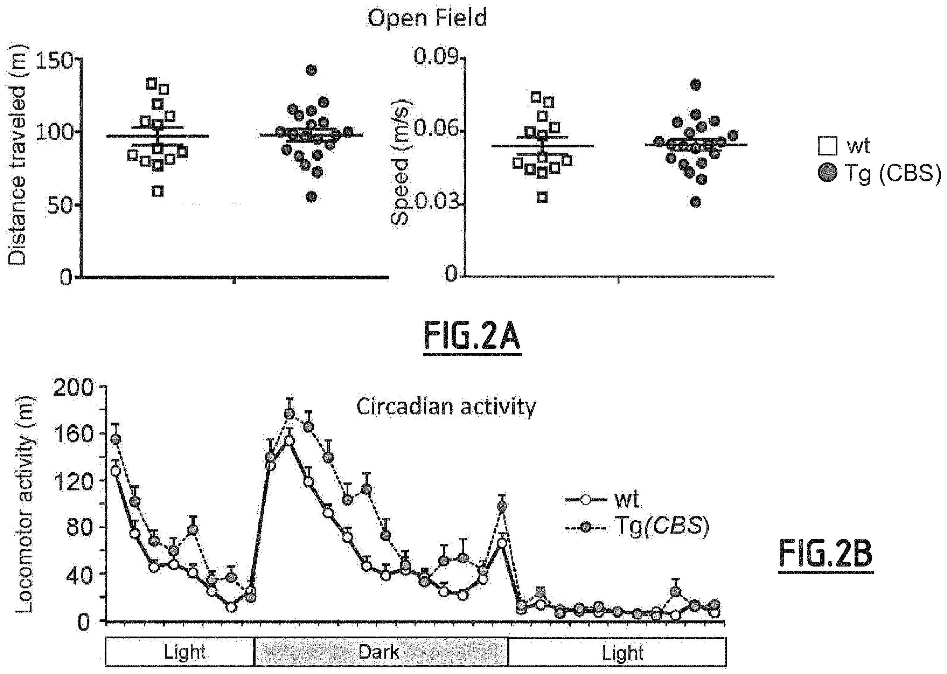

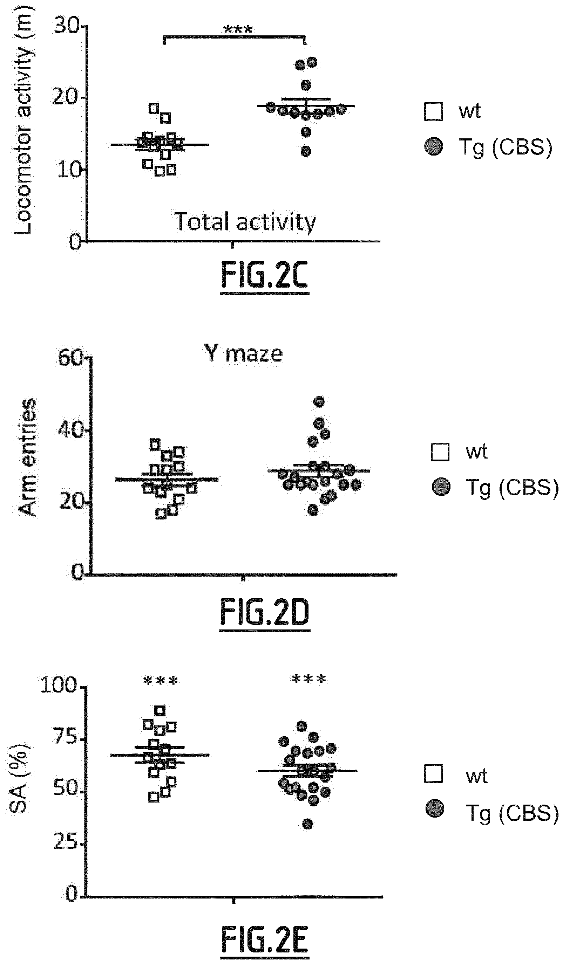

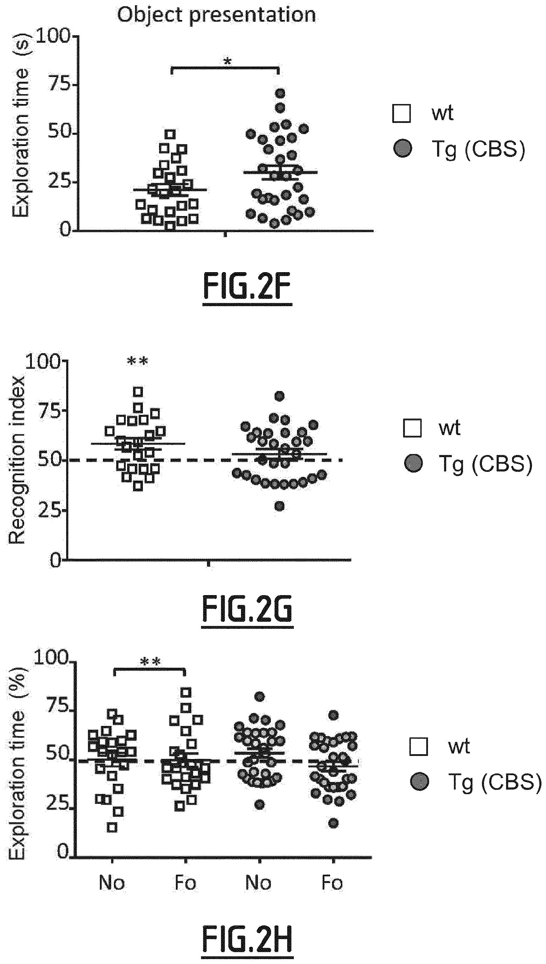

The Tg(*CBS*), a PAC transgenic line encompassing a 60kb fragment with the human *CBS* locus (Butler, C., A. J. Knox, J. Bowersox, S. Forbes, and D. Patterson. 2006. "The production of transgenic mice expressing human cystathionine beta-synthase to study Down syndrome." Behav Genet 36 (3):429-38. doi: 10.1007/s10519-006-9046-y) was used to analyse the impact of the sole increase of *Cbs* dosage on behaviour and cognition. As shown in figure 2A, no difference in locomotor activity was observed during the exploration of a new environment in the open field test between wt and transgenic littermates (Student t-test distance: wt vs Tg(*CBS*)/0 p=0.925; speed wt vs Tg(*CBS*)/0 p=0.925). However, according to the present invention it was found higher circadian activity for isolated individuals (Figure 2C; student t-test wt vs Tg(*CBS*) p<0.001) which results from an increased locomotor activity during the habituation and the dark phase (Figure 2B). In the Y maze (Figures 2D-E), no difference was detected for the number of arm entries and the spontaneous alternation. In the novel object recognition test, (Figures 2F-H) the Tg(*CBS*)/0 animals spent more time sniffing the two identical objects during the presentation phase than their control littermates (Figure 2F; Student t-test wt vs Tg(*CBS*)/0 p=0.05) but were impaired in object recognition as shown by the absence of discrimination between novel and familiar objects for the transgenic mice (Figure 2G: Student paired t-test wt "Fo vs No" p= 0.008; Tg(*CBS*) "Fo vs No" p=0.174) resulting in a recognition index (time on the new object / total time) not significantly different from the 50% chance level, (Figure 2H: one sample t test, significant difference from 50%, wt p= 0.008; Tg(*CBS*)/0 p= 0.174). Consequently the present invention demonstrated that CBS overexpression is sufficient to induce deficit in novel object recognition memory and decreased locomotor activity during dark phase while having no effect during the light phase.

### Example 3 - Cbs overexpression in hippocampal and cortical neurons induces behavioural defects similar to Dp1Yah

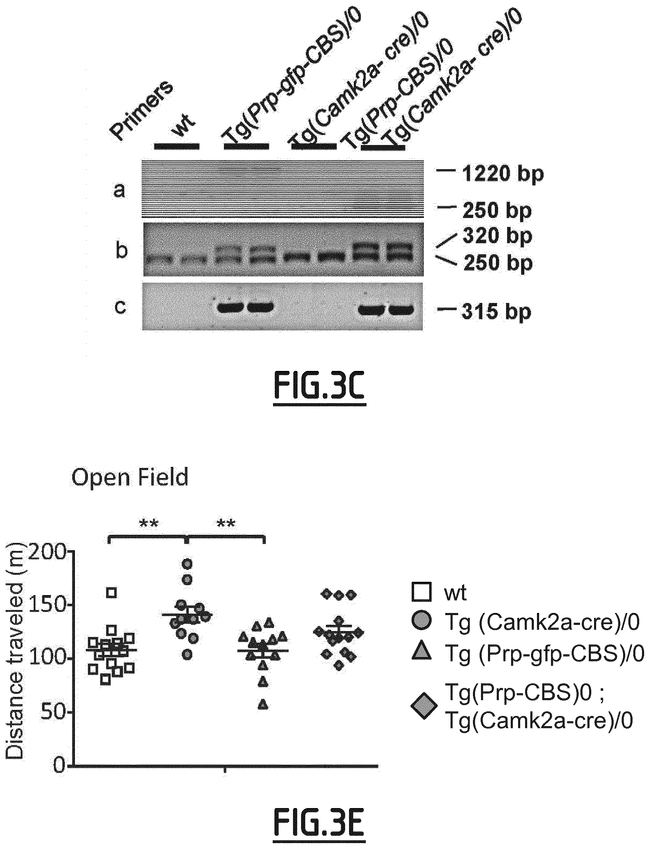

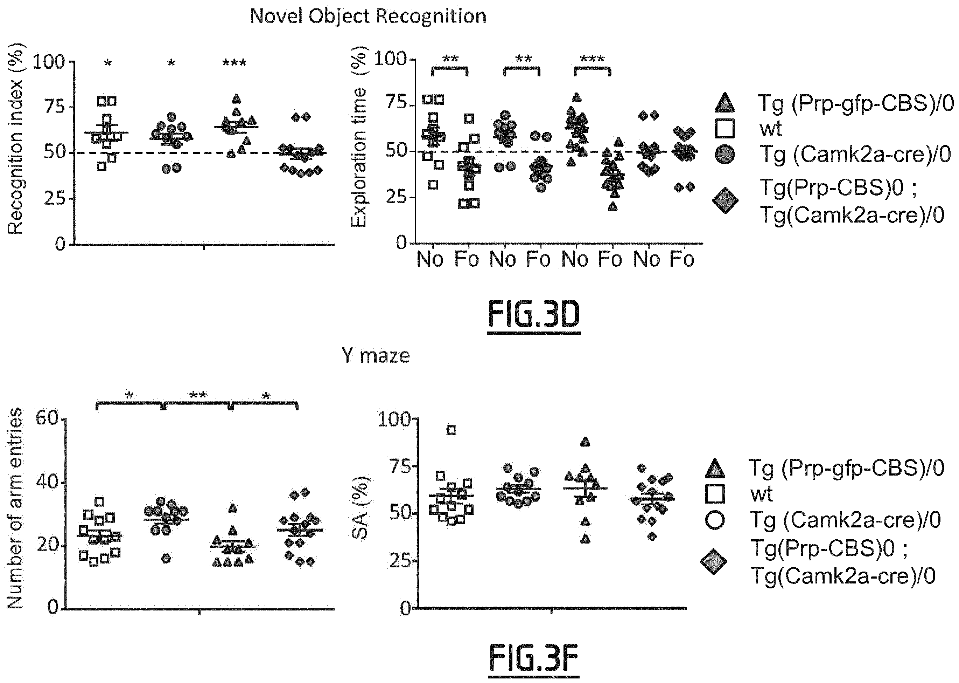

According to the present invention it was checked if the cognitive deficits observed in DS mouse models could be induced by overexpressing *Cbs* mostly in the hippocampal and cortical neurons involved in learning and memory. Hence the Tg(*Prp-gfp-CBS*) mouse strain was engineered in which the human CBS cDNA can be expressed from the Prion promoter after the excision of the gfp cassette flanked by IoxP sites (Figure 3A) and selected one Tg(*Prp-gfp-CBS*) line with a pattern of expression in the anterior part of the adult brain (Figure 3B). The *Tg(Camk2a-cre)* was chosen(Mantamadiotis, T., T. Lemberger, S. C. Bleckmann, H. Kern, O. Kretz, A. Martin Villalba, F. Tronche, C. Kellendonk, D. Gau, J. Kapfhammer, C. Otto, W. Schmid, and G. Schütz. 2002. "Disruption of CREB function in brain leads to neurodegeneration." Nat Genet 31 (1):47-54. doi: 10.1038/ng882), to direct the cre expression in the cortical and hippocampal glutamatergic neurons and the expression of the human *CBS* was verified in different brain regions of the double transgenic (Tg(*Prp-gfp-CBS*)/0;Tg(*Camk2a-cre*)/0)*.* Accoridng to the present invention, expression levels were found comparable to the endogenous murine *Cbs* gene in cerebellum while human CBS was overexpressed in the hippocampus and the cortex (Figure 3C). Littermate animals carrying wt, the two single transgenic constructs and the two transgenes were produced and tested for object recognition. During the test, the control groups, namely wt, Tg(*Prp-gfp-CBS)*/*0* and Tg(*Camk2a-cre*)/0*,* spent more time on the new object (No) than the familiar one (Fo) as expected, while the double transgenic individuals were not able to differentiate the new object from the familiar one as shown by the recognition index or the percentage of exploration time (Figure 3D; Recognition index: One sample t-test: wt p=0.03; Tg(*Camk2a-cre*)/0 p=0.03; Tg(*Prp-gfp-CBS)*/*0* p=0.001; (Tg*(Prp-gfp-CBS*)/0; *Tg*(*Camk2a-cre*)/*0)* p=0.90; exploration time; two ways ANOVA, variables "genotype" and "objects": F(3; 76)= 8.59 with p<0.001; post hoc Tuckey method wt «No vs Fo» p<0,001; Tg(*Camk2a-cre*)/0 «No vs Fo» p=0.001 and Tg(*Prp-gfp-CBS)*/*0* «No vs Fo» p<0.001; (*Tg*(*Prp-gfp-CBS*)/0; *Tg*(*Camk2a-cre)*/0)) «No vs Fo» p=0,861).

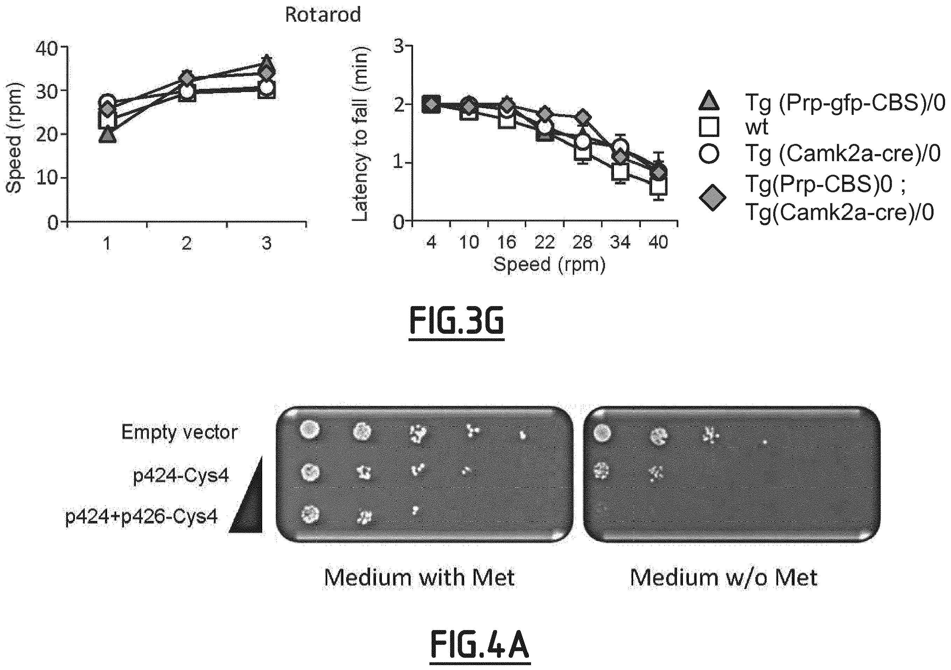

Measurements of the travelled distance in the open field and number of visited arms in the Y maze revealed hyperactivity of the Tg(*Camk2cre*)/0 carrier groups (Figures 3E-F; Openfield: One way ANOVA F(3,49)=4.80 p=0.005; post hoc Holm-Sidak «wt vs Tg(*Camk2-Cre*)/0» unadjusted p=0.002; «Tg(*Prp-gfp-CBS*)/0 vs Tg(*Camk2-Cre*)/0» p=0.003) - Y maze: One way ANOVA F(3,46)=6.04 p=0.001; post hoc Holm-Sidak «wt vs Tg(*Camk2-Cre)*/0» p=0.04; «Tg(*Prp-gfp-CBS*)/0 vs Tg(*Camk2-Cre*)/0» p=0.009; Tg(*Prp-gfp-CBS*)/*0* vs Tg(*Prp-gfp-CBS*)/0;Tg*(Camk2a-cre*)/*0*p=0.04). Like for the Dp1Yah and Tg(*CBS*) animals, we did not found any alteration in the spontaneous alternation in the Y maze test (One way ANOVA: F(3,43)=0.691 p=0.563). All the mice, whatever their genotype, performed equally well during the training session of the rotarod (Figure 3G) (training: repeated measures ANOVA, variables « genotype » and « day », F(3;90)=2.011 p=0.126; test: repeated measures ANOVA, variables « genotype » and « day », F(2;90)=44.783 p<0.001) as well as during the test session with increasing speed (Repeated measures ANOVA, variables « genotype » and « speed », F(18;322)=0.631 p=0.875). Thus, as expected from the role of the cerebellum in locomotor coordination, the overdose of CBS restricted to cortical and hippocampal neurons did not interfere with the locomotor activity.

Hence, overexpression of CBS is necessary and sufficient to induce object memory defect in a 1h retention test with limited impact on other phenotypes. As such, *CBS* is a new gene whose overdosage alters cognition in DS mouse models and as a consequence is likely to contribute to DS phenotypes.

### Example 4 - Identification of drugs that suppress the effects of Cys4/CBS overexpression both in yeast and mouse

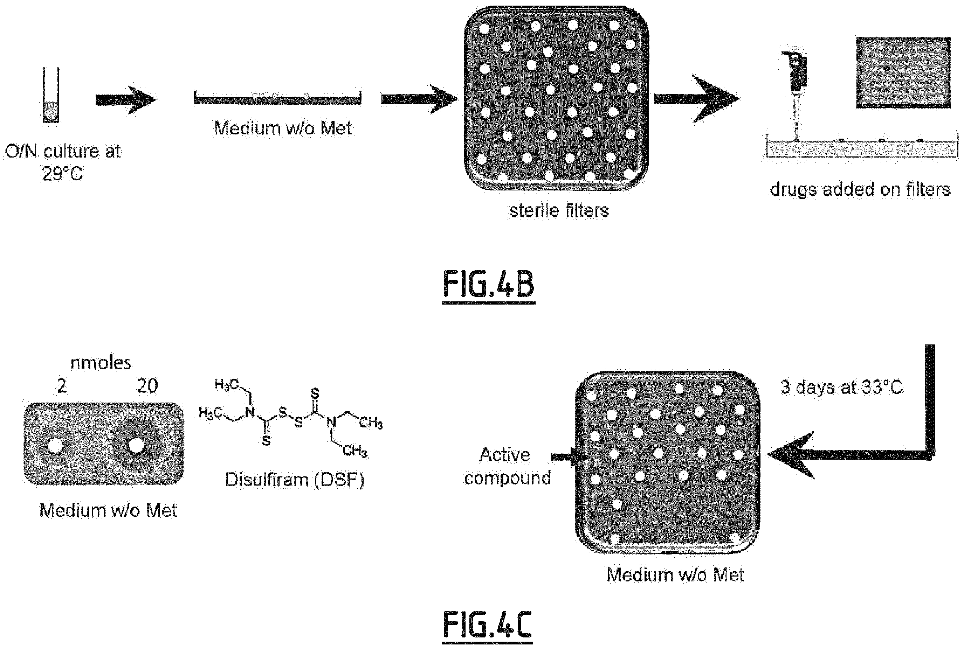

A few studies have reported the identification of CBS inhibitors but most of them were based on *in vitro* assays using a recombinant CBS enzyme as a drug target and led to the isolation of inhibitors with relatively low potency and limited selectivity, hence leading to the idea that CBS may be an undruggable enzyme. Therefore according to the present invention, it was decided to orient toward an *in cellulo* phenotype-based assay that would allow screening drugs that interfere with the phenotypical consequences of CBS overexpression and thereby that do not necessarily directly target the CBS enzyme. The budding yeast *Saccharomyces cerevisiae* contains a functional homolog of CBS (Cys4p, encoded by the *CYS4* gene) and has been shown to be a relevant system to model pathophysiological mechanisms involved in a number of human disorders and to perform chemobiological approaches that aim at identifying both drugs and new therapeutic targets. According to the present invention, it was decided to create a yeast model in which the phenotypical consequences of CBS overexpression may be easily and conveniently monitored in order to get a potential *in cellulo* high throughput drug screening procedure. According to the present invention, it was reasoned that if CBS was overexpressed at a sufficient level, this should lead to a decreased intracellular level of methionine, similarly to what was shown in patients, and therefore that yeast cells would become methionine auxotroph and thereby unable (or only weakly able) to grow on methionine-free minimal media. As the human CBS protein is not very stable in yeast cells and therefore cannot be expressed at high levels, it was decided according to the present invention to overexpress Cys4p (encoded by the *CYS4* gene), the CBS homolog in *S. cerevisiae.* Cys4p presents the same domains and domain organization than CBS apart from the N-terminal heme-binding domain which is absent in the yeast protein (Kruger, W. D., and D. R. Cox. 1994. "A yeast system for expression of human cystathionine beta-synthase: structural and functional conservation of the human and yeast genes." Proc Natl Acad Sci U S A 91 (14):6614-8). To get a degree of methionine auxotrophy sufficient to allow an efficient screening, *CYS4* was expressed from the strong constitutive *GPD* promoter from two different high copy number 2 µ vectors (each present at ∼50 copies per cell) and supplement the growth medium with serine, which is one of the Cys4p/CBS substrates that could otherwise become limiting upon *CYS4* overexpression (Figure 4A). Using this model, ≈ 2200 compounds were tested from 3 different chemical libraries consisting mainly of repurposed drugs for their ability to suppress the methionine auxotrophy induced by Cys4p overexpression. Briefly, yeast cells overexpressing Cys4 were spread on a solid agar-based methionine-free minimal medium. Then filters were put on the agar surface and different drugs from chemical libraries added on each filters. After 3 days of incubation at 33°c, active compounds were identified by a halo of restored/enhanced growth around the filter on which they were loaded (Figure 4B). The advantage of this method is that, in one simple experiment, it allows numerous compounds to be tested across a large range of concentrations due to the diffusion of the molecule in the medium surrounding the filter onto which it was deposited. This design drastically improves the sensitivity of the screen because the screened compounds can be toxic at high concentrations whereas being active at subtoxic concentrations. four different compounds were identified, among which disulfiram (DSF, Figure 4C).

Next according to the present invention, it was tested if DSF was able to restore the object recognition of the mouse model overexpressing human *CBS.* Three independent cohorts of Tg(*CBS*) and control littermates were treated with DSF (10mg/kg/day) for 10 days before being tested for the novel object recognition. As shown in figure 4D, DSF-treated transgenic animals were restored in the novel object recognition paradigm whereas non treated mutant animals were still not able to discriminate the new versus the familiar object. Interestingly the DSF-treated wt individuals were no more able to perform the discrimination while the vehicle-treated controls were able to do so (Student paired t-test: vehicle treated wt «No vs Fo» p=0,006; DSF treated wt «No vs Fo» p=0.11 and vehicle treated Tg(CBS) «No vs Fo» p=0.59; DSF treated Tg(*CBS*) «No vs Fo» p=0,05). This goes in line with the fact that loss-of-function mutations in CBS also leads to cognitive defects as observed in homocystinuria patients. Hence, this latter result confirm that DSF does affect CBS activity, directly or indirectly. Altogether these results confirm that the phenotypical consequences of the overexpression of CBS could be targeted by drugs to restore some of the cognitive performance altered in DS models. They also emphasize that the inhibition of CBS, direct or indirect, should be mild and only partial as a strong inhibition may be detrimental as illustrated by the cognitive dysfunction observed in homocystinuria and here in wt mice treated with DSF.

### Example 5 - Genotype identification

The genotypes of the different alleles used in the study were assigned after PCR amplification on isolated genomic DNA (95°C / 5min; 35 cyles (95°C / 45sec, specific Tm / 45sec, 68°C / 1min), 68° 5min) with the use of the specific pairs of primers listed in the supplementary table 1. The Dp1Yah genotype was determined as described previously (Pereira, P. L., L. Magnol, I. Sahun, V. Brault, A. Duchon, P. Prandini, A. Gruart, J. C. Bizot, B. Chadefaux-Vekemans, S. Deutsch, F. Trovero, J. M. Delgado-Garcia, S. E. Antonarakis, M. Dierssen, and Y. Herault. 2009. "A new mouse model for the trisomy of the Abcg1-U2af1 region reveals the complexity of the combinatorial genetic code of down syndrome." Human Molecular Genetics 18 (24):4756-4769. doi: 10.1093/hmg/ddp438).

### Example 6 - Behavioural protocols

**6.1. The circadian activity** was measured to assess spontaneous activity and feeding behaviour over the complete light/dark cycle. Testing was performed in individual cages (11 x 21 x 18 cm) fitted with infrared captors linked to an electronic interface (Imetronic, Pessac, France) that provided automated measures of position and locomotor activity. Mice were put into cages at 11 am on the first day and removed on the next day at 7 pm. The light cycle was set as 12 h light and 12 h dark (light on at 7 am). The 32 hours of testing were divided into three different phases: the habituation phase (from 11 am to 7 pm on the first day); the night/dark phase (from 7 pm on the first day to 7 am on the second day); and the day/light phase (from 7 am to 7 pm on the second day). Feeding behaviour was evaluated using an automated lickometer (Imetronic, Pessac, France) and a 20 mg pellet feeder (Test Diet, Hoffman La-Roche).

For the **openfield** paradigm, mice were tested in a 55 cm diameter white round box and mouse activity was recorded with a video tracking system (Ethovision, Noldus, France) during a unique session of 30 min. The arenas were placed in a room homogeneously illuminated at 50 Lux. Each mouse was placed at the periphery of the open-field and allowed to explore the apparatus freely for 30 min with the experimenter out of the animal's sight. The distance travelled and time spent in the central and peripheral regions were recorded over the test session. The number of entries and the percentage of time spent in the centre area are used as indexes of emotionality/anxiety.

**6.2. The object recognition task** was performed in the same conditions as the open-field (see above). The objects to be discriminated were a glass marble (2.5 cm diameter) and a plastic dice (2 cm). The animals were first habituated to the open-field for 30 min. The next day, they were submitted to a 10 min acquisition trial during which they were placed in the open-field in the presence of two similar objects A (marble or dice). The time the animal took to explore the object A (when the animal's snout was directed towards the object at a distance ≤ 1 cm) was recorded manually. A 10 min retention trial was performed 1 h later. During this trial, one of the familiar objects was changed for a new one in the open-field, and the times tA and tB that the animal took to explore the two objects were recorded. We used a minimal time of three seconds to select animals during the acquisition trial,and the retention trial. The exploration index for object B was defined as (tB / (tA + tB)) x100. Memory was defined by the percentage of time animals spent investigating the novel object statistically different from the chance (50%). We also noted activity parameters such as speed, distance and the time spent in the centre and at the periphery of the apparatus. To control for odour cues, the arena and the objects were thoroughly cleaned with 50% ethanol, dried, and ventilated for a few minutes between sessions.

**6.3. The Y-maze** evaluates the immediate working memory. It is composed of three identical arms but with three different motifs on the wall. The animals were placed in the centre of the apparatus, at the arms intersection, and were left free to explore the maze during 6 minutes. We recorded the sequence of visited arms. A visit is defined as a complete entrance with the 4 paws in the arm. After the session, we calculated a memory index with the percentage of spontaneous alternation: A= (number of 3 consecutive different visits / total of visited arms minus 2) x 100. **64. The Morris water maze** was used to test spatial learning and memory. The water maze is a circular pool (150-cm diameter, 60-cm height) filled with 40 cm with water maintained at 20°C-22°C, made opaque using a white aqueous emulsion (Acusol OP 301 opacifier). The surface was split into 4 quadrants: South-East (SE), North-West (NW), North-East (NE), South-West (SW). The escape platform, made of rough plastic, was submerged 1 cm below the water's surface. Experiments were performed to study reference memory through a spatial search strategy that involved finding the hidden platform. The spatial memory session consisted of a 6-day (J1 to J6) learning phase with four 90-second trials per day. Each trial started with mice facing the interior wall of the pool and ended when they climbed onto the platform located on the SE quadrant or after a maximum searching time of 90 sec. The starting position was changed pseudo-randomly between trials. Mice were left undisturbed in their home cage for 90-min inter-trial intervals. On the seventh day, mice were given the 60-sec probe test in which the platform had been removed. The distance traveled in each quadrant (NW, NE, SW, SE) was recorded as well as the time spent in the target quadrant.

**6.5. Motor coordination** was assessed with the rotarod during the training and the test phases. The evaluation criterion is the time that the mouse spends on the rotating rod before falling. The apparatus (Bioseb, France) is made of a rotating bar of 5 cm diameter on which mice are placed facing the direction of rotation. The test was divided in 3 phases: a habituation phase of 2 min on the road at 4 rpm; a training phase in which each mouse had 4 trials per day, on 3 consecutive days. This step is an accelerated mode (ARR) from 4 to 40 rpm spread on 5 minutes. The last phase is based on constant speed sessions (FSRR), the 4th day of the test; it consisted of 7 trials of 2 minutes, each trial at one selected speed (4, 10, 16, 22, 28, 34 and 40 rpm) and this procedure was repeated twice. For each trial of the four days (training + test), the time until the mouse fell off the rod was recorded as well as the corresponding speed. This protocol is adapted from (Duchon et al., 2011).

**Supplementary table 1: Primers/probes used for genotyping.**

| Mouse lines | Primers | SEQ ID NO: | Sequences5'-3' | Tm (°C) | Size of Amplicon |
|---|---|---|---|---|---|
| Tg(*Camk2a-cre*) | Cre-fo | 5 | CTGCATTACCGGTCGATGCA | 57 | 355 bp |
| | Cre-re | 6 | ACGTTCACCGGCATCAACGT | | |
| Tg(*Prp-gfp-CBS*) | Myo-fo | 7 | ATCCTGGAGATGGACCACTTG G | 58 | 250 bp |
| | Myo-re | 8 | GATGAGTTTGGACAAACCACA ACTAGAATGC | | |
| | hCBS-fo (b) | 9 | CTCCCGGGACGAATTCGCAC | 58 | 320 bp |
| | hCBS-re (b) | 10 | TCTTGTTGATTCTGACCATAG GGGTGTC | | |
| | pA-fo (c) | 11 | ATCCTGGAGATGGACCACTTC GC | 58 | 315 bp |
| | pA-re (c) | 12 | GATGAGTTTGGACAAACCACA ACTAGAATGC | | |
| | Tg(Prp-Gfp-CBS)-fo (a) | 13 | GCATTCTGCCTTCCTAGTGG | 58 | 1159 bp* |
| | Tg(Prp-Gfp-CBS)-re (a) | 14 | GCTCCTTGGCTTCCTTATCC | | 247 bp** |
| *Cbs^{tm1Unc}* | *Cbs*-commun-fo | 15 | GGTCTGGAATTCACTATGTAG C | 62 | |
| | *Cbs*-wt-re | 16 | AAGAGCCCAGCAGAATGAACA | | 260 bp^{§} |
| | *Cbs*-tm1 Unc-re | 17 | GAGGTCGACGGTATCGATA | | 190 bp^{§§} |
| Tg(*CBS*) | Tg(*CBS*)-fo | 18 | TGTTTCACCAAGGAA ATATTGAGA | 60 | 264 bp |
| | Tg(*CBS*)-re | 19 | AAATGCCGCTGATTGTTCAC | | |
| Tg(*Dyrk1a*) | Tg(*Dyrk1a*)-fo | 20 | TGG GCC AAG CAG TTA GGA GTT T | 60 | 230 bp |
| | Tg(*Dyrk1a*)-re | 21 | CCA TGA TTA CGC CAA GCT ATT TAG G | | |
| | m*Fez*-fo | 22 | GCT TCG GGA GCA GGT ACC CTA T | 60 | 185 bp |
| | M*Fez*-re | 23 | CAA GAT CTG AGG CTC GCC AAG T | | |

| | | | | | |
|---|---|---|---|---|---|
| (fo: forward; re: reverse; * before and **after Cre excision; ^{§} wt and ^{§§} mutant *Cbs* alleles) | | | | | |

### DISCUSSION

According to the invention, it was demonstrated that the genetic overdosage of *Cbs* is necessary and sufficient to induce defective novel object recognition in 3 different types of DS models. CBS overdosage is certainly the main driver of the learning and memory phenotypes detected previously in DS models for the Mmu17 region but it cannot be ruled out the possibility that one or more other gene(s) contribute with *Cbs* to the phenotype. Previous analysis of CBS overdosage with the same transgenic line Tg(*CBS*) on the FVB/N genetic background showed no change in fear learning task and locomotor activity but increased LTP-dependent synaptic plasticity; a phenomenon also detected *in vitro* and *in vivo* in other DS models where *Cbs* is trisomic in the C57BL/6J genetic background. Nevertheless no positive effect on cognition is associated with increase CBS dosage. Instead the overdosage of CBS always impairs the hippocampal-dependent novel object recognition test suggesting that increased synaptic plasticity found in *Cbs* trisomic models may alter synaptic functions. Increased synaptic plasticity could occur via increased H₂S as it has been shown that H₂S facilitates LTP by stimulating the post-synaptic NMDA receptors. Moreover, a role of H₂S has been foreseen in calcium homeostasis regulation which is also crucial for neuronal synaptic plasticity.

DSF was isolated from a drug screening performed in yeast cells overexpressing CBS homolog Cys4p and looking for drugs counteracting its effect on methionine auxotrophy. Although DSF has been first identified as an inhibitor of mitochondrial aldehyde dehydrogenase (ALDH), it is a relatively nontoxic substance, which has been on the market for more than 40 years to support the treatment of chronic alcoholism by producing an acute sensitivity to ethanol, thanks to its ability to inhibit aldehyde dehydrogenases, thus leading to an accumulation of acetaldehyde in blood when alcohol is ingested. As acetaldehyde is responsible for many of the unpleasant effects that follow ingestion of large quantities of alcohol ("hangover"), DSF treatment discourages the patients to sustain a regular alcohol consumption by exacerbating and accelerating its unpleasant side effects. Preliminary data according to the invention about the mechanism of action of DSF suggest that this molecule may not directly inhibit CBS enzymatic activity but probably rather acts on the cellular consequences of CBS overexpression. The assay used for the screening, in principle, leads to the isolation of drugs acting both directly or not on CBS/Cys4. This latter point is of importance given that CBS may not be a druggable target enzyme. And indeed, at present, it is unknown if the DSF is acting directly or indirectly on CBS but the function altered by CBS overdosage, whatever it is, is certainly conserved and similarly sensitive to DSF treatment in both yeast and mouse. Of note, upon absorption DSF is rapidly reduced to diethyldithiocarbamate (DDC), which then reacts with thiol groups. Both DSF and DCC are potent copper chelators, thereby possibly affecting the activity of copper-dependent enzymes such as monooxygenases, the Cu-Zn superoxide dismutase, amine oxidase, ADN methyltransferases and cytochrome oxidase. As a result, DSF has been shown to affect various cellular processes such as cocaine metabolism and catecholamine synthesis, and proteasome inhibition, and is thus under study for multiple clinical applications that include struggle against alcohol addiction, cancer chemotherapy, treatment of copper-related disorders and anti-viral treatment for hepatitis C and Human Immunodeficiency Virus.

In the present invention, a new possible clinical application of DSF in DS cognition was discovered through its effect on CBS overexpression. CBS clearly represents a new relevant therapeutic target for improving DS cognition and DSF, as such, opens new therapeutic avenues in DS patients.

The present invention also demonstrated that CBS interacts genetically with *Dyrk1a,* a well-known therapeutic target for DS. Mutual relationships between DYRK1A and CBS were shown previously, with decreased DYRK1A protein observed in the liver and increased expression observed in the brain of *Cbs*^{+/-} mice, while overexpression (or under-expression) of DYRK1A induce accumulation (or reduction) of CBS expression in the liver. In order to explore the genetic interactions between DYRK1A and CBS, *Dyrk1a* was overexpressed in the Dp1 Yah context by combining the *Tg*(*Dyrk1a*) and the Dp1 Yah mice. Surprisingly, this experiment restored the object recognition deficit observed in the Dp1Yah mouse model but neither the increased locomotor activity in the open-field or the Y maze, nor the working and spatial memory deficits. Thus the compensation is restricted to a specific cognitive function, recognition memory, which is defective in both TgDyrk1a and Dp1Yah models. Why this dosage effect is restricted to recognition memory remains speculative. It can be hypothesized that *Cbs* and *Dyrk1a* overdosage only interact in specific regions of the adult brain involved in object discrimination explaining why the increased locomotor activity and the working and visuo-spatial phenotypes induced in Tg(*Dyrk1a*) animals are not affected. Alternatively, objects recognition deficit is likely to result from an impact of DYRK1A on adult brain function while the other phenotypes are the result of an impact during earlier stage of brain development. On the one hand, object recognition has been shown to require undamaged hippocampal perforant path connecting entho/perirhinal cortex with the dentate gyrus for long retention intervals (> 15 min) in rat. On the other hand, synaptic exchanges between the median prefrontal cortex (mPFC) and the hippocampus seems to be sufficient to support the processing of short-term memory such as working memory observed in the Y maze and hyperactivity is associated with the prefrontal cortex, basal ganglia and cerebellum. Moreover, long-term recognition memory has been shown to appear in the rat at weaning (post-natal day 21 in the mouse), a period corresponding to the end of neurogenesis and synaptogenesis in the dentate of the hippocampus, and reflecting the general observation of 'infantile amnesia' observed on long-term memory tasks but not on short-term memory ability.

The present invention goes farther than the demonstration that the Hsa21 homologous region on the Mmu17 is a key determinant cognitive deficits in DS mouse models. The present invention showed that CBS is a key gene for DS related phenotypes in mice with the other homologous interval *Cbr3-Fam3b* located on Mmu16, encompassing *Dyrk1a.* Also, it was considered that in people with DS, both genes are trisomic and thus the recognition memory deficit observed in DS persons and in the complete T21 mouse model certainly depends not only on the interplay between DYRK1A and CBS but also on interaction with other Hsa21 genes that may affect different pathways or different parts of the brain.

DYRK1A is the main driver of defects in DS mouse models for the homologous region to Hsa21 located on Mmu16. Based on study done in DS models for the Mmu16 homologous region, DYRK1A has been selected as a drug target. As reported previously, a treatment with epigallocatechin-3-gallate (EGCG), an inhibitor of DYRK1A kinase activity, can restore some cognitive aspects found altered in people with DS but the gain was limited. Nevertheless results according to the present invention, by adding CBS to the limited number of DS therapeutic targets, may improve the efficiency of DS treatment, in particular by combining multiple therapies for improving the life of DS patients. Finally, an important point to emphasize is that, for DYRK1A as well as for CBS, both loss of function mutations and overdosage lead to intellectual deficiencies. This is important to keep in mind when considering pharmacological intervention that aims at inhibiting one or the other, or both, of these enzymes. Therefore, drug treatment that lead to only a mild inhibition of CBS and/or DYRK1A should be favoured.

### LEGENDS TO FIGURES

**Figure** 1: **The Dp1Yah phenotypes are dependent on *Cbs* dosage.**
   Dp1Yah trisomic mice (n=23) were compared with Dp1Yah carrying a KO of *Cbs (Dp1Yah*/*Cbs^{tm1Unc},* n=21)), *Cbs*^{*tm1Unc*/+} (n=23) and wt littermates (n=29). Animals were analysed for the open field (A), the Y maze (B) and the novel object recognition (D) in two independent cohorts; the rotarod (C) was assessed on one cohort with wt (n=18) *Cbs*^{*tm1Unc*/+} (n=15), Dp1Yah (n=15) and Dp1Yah/*Cbs^{tm1Unc}* (n=10) littermates. (A) Distance travelled and medium speed during the 30min of the test were increased in the *Dp1Yah*/*Cbs^{tm1Unc}* compared to the wild type genotype. (B) Increased exploration activity was confirmed for the *Dp1Yah*/*Cbs^{tm1Unc}* mice compared to control littermates in the Y maze while spontaneous alternation was not affected. (C) During the training session (left panel), the Dp1Yah mice were not able to improve their performance on the rotarod by increasing the maximum of speed before they fall from the rod compared to the other genotype. Nevertheless no change was observed between individuals with the four genotypes during the test phase (right panel). (D) The exploration time in the first session of the novel object recognition (left upper panel) was not statistically different in the four genotypes but during the recognition phase, after 10 min of retention, the recognition index (right upper panel; time spent on the new object / total time of exploration) was clearly lower in Dp1Yah mice as compared to the other genotypes and not statistically different from chance (50%). Accordingly the exploration time (left lower panel) spent by the *Dp1Yah*/*Cbs^{tm1Unc}* mice to explore the object showed that they were able to differentiate the novel (No) versus the familiar (Fo) object while the Dp1Yah were not. Data are represented as one point per individual tested and the mean of the group. (Values represent means + S.E.M. *P<0.05, **P<0.01, ***P<0.001).
**Figure 2****: Transgenic mice overexpressing human CBS display DS-related behaviour phenotypes.**
   Wt (n=13) and Tg(CBS)/0 littermates (n=17), hemizygotes for a human PAC containing the *CBS* gene, were tested for open field (A), circadian actimetry (B,C), Y maze (D-E) and novel object recognition (F,G and H). No phenotype was found in the Tg during the exploration of a new environment in the open field in the total distance travelled (left) and the speed (right) but increased activity was observed during home cage monitoring over a light-dark-light cycle (B) with an increase of the distance travelled (C). In the Y maze (E), Tg(*CBS*)/0 animals displayed altered spontaneous alternation with no change in the number of arm entries (D). In the novel object recognition (F), Tg(*Cbs*)/0 mice displayed similar exploration activity compared to wt littermates but they do not discriminate the novel versus the familiar object when looking at the discrimination index (G) and the percentage of exploration time for both objects (H). (Values represent means + S.E.M. *P<0.05, **P<0.01, ***P<0.001).
**Figure 3****: Selective overexpression of *hCBS* in the glutamatergic neurons leads to impaired object recognition and altered locomotor activity.**
   (A) a conditional transgene Tg(*Prp-gfp-CBS)* was designed to overexpress the human CBS cDNA from the murine Prion promoter after the deletion of an interrupting GFP-coding cassette flanked by IoxP sites. The GFP allowed to select one line that lead to expression in the anterior part of the brain (B). When Cre is expressed from the Tg(Camk2-Cre) transgene, the deletion can be monitored in the brain of the animals (C) and the overexpression of hCBS mRNA is detected in different part of the brain of the Tg(Camk2-Cre)/0;Tg(*Prp*-*gfp*-*CBS*)/0 animals (Hs, orange bar, B) with no change in the endogeneous murine CBS without Cre expression detected in wt animals (Mm, white bar B) or Tg(Camk2-Cre)/0;Tg(*Prp*-*gfp*-*CBS*)/0 animals (Mm, orange bar, B). Wt (n=13), Tg(Camk2-Cre)/0 (n=11), Tg(*Prp-gfp-CBS)*/0 (n=12), and Tg(Camk2-Cre)/0;Tg(*Prp-gfp-CBS*)/0 (n=14) littermates were evaluated through for object discrimination (D), open field (E), Y maze (F), rotarod (G). Mice overexpressing hCBS in the glutamatergic neurons were unable to discriminate the novel versus the familiar object as compared to the other control genotypes (D). Tg(Camk2-Cre)/0 mice displayed an enhanced locomotor activity in the open field but no change was detected in the control, wt and Tg(*Prp-gfp-CBS*)/0*,* or in double transgenic animals (E). In the Y maze animals carrying the Tg(*Prp-gfp-CBS)*/*0* or the activated form, Tg(Camk2-Cre)/0;Tg(*Prp*-*gfp*-*CBS*)/0, displayed reduced exploration with a lower number of arm entries but no change in the spontaneous alternation (F). No phenotypes was altered in the rotarod test with similar progress during the learning and the test phases (G). (Values represent means + S.E.M. *P<0.05, **P<0.01, ***P<0.001)
**Figure 4****. Pharmacological intervention to suppress the consequence of CBS overexpression in yeast (A, B and C) and mouse (D).** Development of a yeast screening assay based on CYS4-overexpressing cells and identification of DSF as able to suppress methionine auxotrophy induced by *CYS4* overexpression. The sensitivity of the strain to the absence of methionine in the medium was evidenced by serial dilutions of a yeast strain expressing different levels of Cys4p (A). For the drug screening, the yeast strain overexpressing *CYS4* from both p424 & p426 2 µ plasmids was spread on a square Petri plate containing solid agar-based methionine-free medium. DMSO was used as a negative control and added to the upper right filter and methionine, the positive control, was deposited on the bottom left filter (B). At the remaining positions, individual compounds from the chemical libraries were added, and plates were incubated for 3 d at 33 °C. The dose-dependent effect of DSF on *CYS4*-overexpressing cells is shown, and its molecular structure is depicted (C). Note that DSF is toxic at high concentrations (close to the filter) whereas it becomes active at sub-toxic concentrations. To test DSF in mice, a treatment was done on Tg(*CBS*) cohort starting at D1 and ending at D10 (D). Each groups received a daily dose of 10mg/kg/day of DSF for 10 days followed by an open field paradigm (D10) with the object recognition test performed on D11 (with one hour of retention time). The graph at the bottom showed the percentage of time spent on the novel versus the familiar object during the tests. The vehicule-treated wt mice were able to distinguish both objects as the the DSF-treated Tg(*CBS*) animals. On the contrary non-treated transgenic animals were not able to do so and the DSF-treated wt animals were impaired in the test confirming that the drug affects CBS activity *in vivo* (Values represent means + S.E.M. *P<0.05, **P<0.01, ***P<0.001).

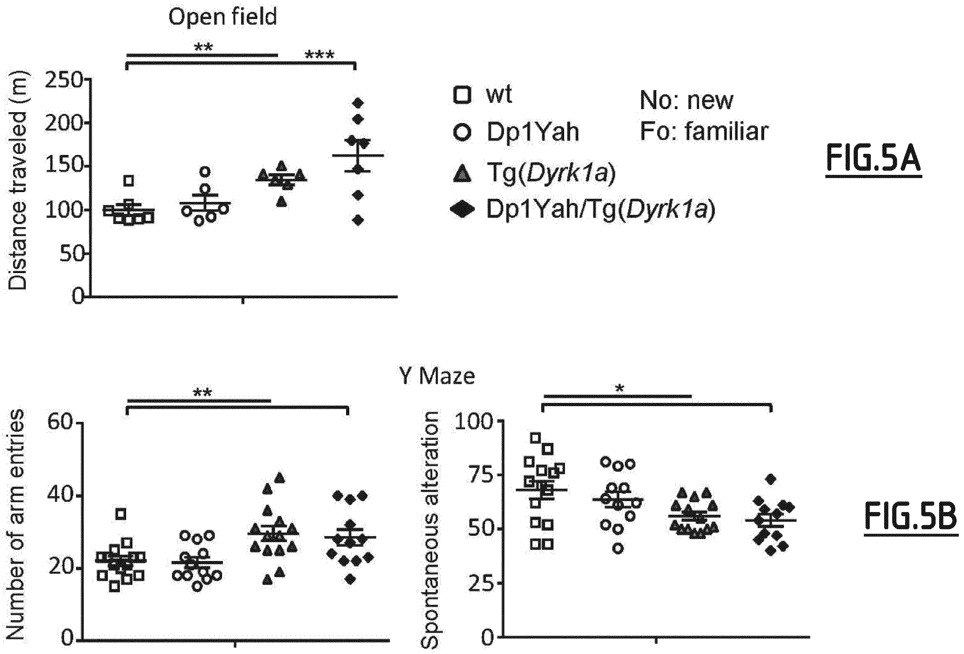

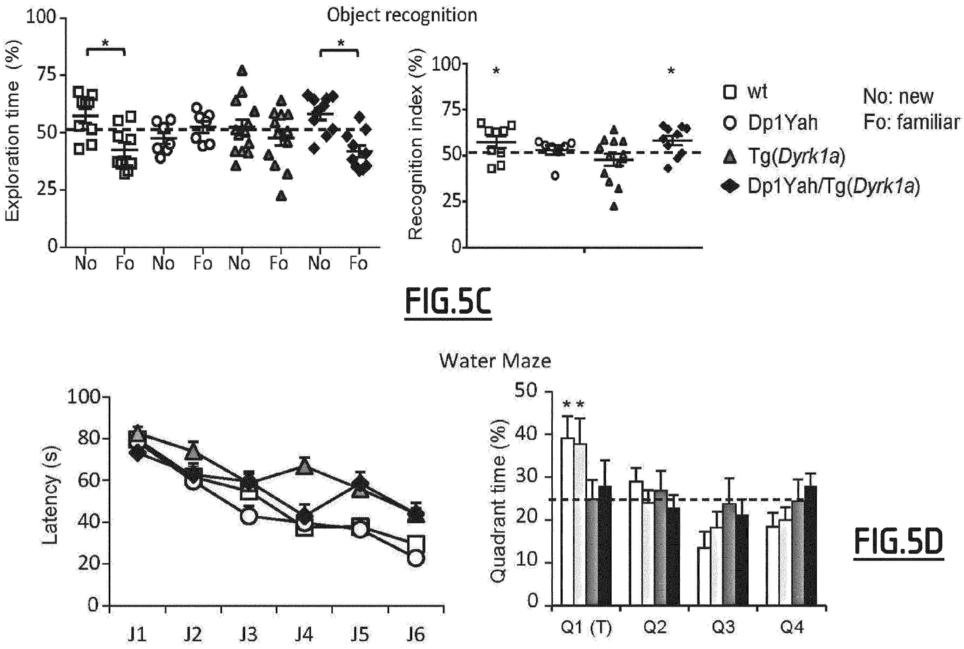

**Figure 5****: CBS and DYRK1A overdosages interact for controlling behaviour and cognition**
   Behavioural and cognitive analysis of transgenic animals overexpressing *Cbs* and *Dyrk1a* (14 wt, 15 *Tg(Dyrkla),* 13 Dp1Yah and 13 Dp1Yah/Tg(*Dyrk1a*)) mutant mice in the open field (A), the Y maze (B), the object recognition (C) and the Morris water maze (D). Increased activity in the open field (A) and in the number of arm entries in the Y maze (B) were found in the *Tg*(*Dyrk1a*) and Dp1Yah/Tg(*Dyrk1a*) animals with also reduced spontaneous alternation in the Y maze (B). Both the Dp1Yah and Dp1Yah/Tg(*Dyrk1a*)) mutant mice were impaired in object recognition (C) but the double mutant animals showed restored object discrimination similar to wt littermates. The *Tg*(*Dyrk1a*) and Dp1Yah/Tg(*Dyrk1a*) animals displayed delayed learning in the Morris water maze with no memory of the platform location in the probe test compared to Dp1Yah and wt littermates (D). (Values represent means + S.E.M. *P<0.05, **P<0.01, ***P<0.001)

## Claims

1. A non human eukaryote cell model wherein said eukaryote cell overexpresses Cystathionine beta-synthase (EC 4.2.1.22) and/or a coding DNA thereof.

2. The eukaryote cell model according to claim 1, wherein said Cystathionine beta-synthase coding gene is Cystathionine b-synthase gene (*CYS4*) (Systematic Name *YGR155W* ; SGD ID SGD:S000003387) or an homologue gene overexpressing said Cystathionine beta-synthase.

3. The eukaryote cell model according to claim 1 or 2, wherein said eukaryote cell is a yeast.

4. The eukaryote cell model according to any one of claims 1 to 3, wherein said eukaryote cell is *Saccharomyces cerevisiae.*

5. A method for screening molecules or compositions, wherein said method comprises screening said molecules or compositions with an eukaryote cell model as defined in any one of claims 1 to 4.

6. The method according to claim 7, wherein said method is for screening molecules or compositions for the treatment of intellectual disability in Down syndrome (DS).

7. The method according to claim 7 or 8, wherein said method comprising detecting molecules or compositions suppressing a methionine auxotrophy induced by Cys4p overexpression.

8. A molecule counteracting at least one effect and/or consequence of overexpression of Cystathionine beta-synthase (EC 4.2.1.22) and/or of a coding DNA thereof, for use in a treatment of intellectual disability in Down syndrome (DS).

9. The molecule according to claim 8, wherein said Cystathionine beta-synthase is human Cystathionine Beta Synthase protein (CBS) and/or a functional eukarotic protein equivalent thereof, for use in a treatment of intellectual disability in Down syndrome (DS).

10. The molecule according to claim 8, wherein said DNA coding for Cystathionine beta-synthase is human Cystathionine Beta Synthase gene (Cbs) and/or a functional eukarotic gene equivalent thereof, for use in a treatment of intellectual disability in Down syndrome (DS).

11. The molecule according to claim 9, wherein said molecule suppresses a methionine auxotrophy induced by *CYS4* overexpression in an eukaryote cell model as defined in any one of claims 1 to 4, for example according to a method as defined in any one of claims 5 to 8.

12. The molecule according to any one of claims 8 to 11, wherein said molecule is selected from the group consisting of Zn ionophore molecules.

13. A molecule selected from the group consisting of:
a thiocarbamate, for example disulfiram (1-(diethylthiocarbamoyldisulfanyl)-N,N-diethyl-methanethioamide - CAS Number 97-77-8),
a 8-hydroxyquinoline, preferably a 5,7-subsituted-8-hydroxyquinolines, for example clioquinol (iodochlorhydroxyquin - CAS Number 130-26-7), chloroxine (5,7-Dichloro-8-hydroxyquinoline - CAS Number 773-76-2),
sodium pyrithione (1-Hydroxy-2(1H)-pyridinethione - CAS number 1121-30-8 (thione) or 2-pyridinethiol 1-oxide - CAS number 1121-31-9 (thiol),
any salt thereof, preferably a Zn salt thereof, for example Zn pyrithione,
any prodrug thereof,
and any combination thereof,
for use in a treatment of intellectual disability in Down syndrome (DS).

14. The molecule according to claim 13, wherein said molecule counteracts at least one effect and/or consequence of overexpression of Cystathionine beta-synthase (EC 4.2.1.22) and/or of a coding DNA thereof.

15. The molecule according to any one of claims 8 to 14, wherein said molecule is administered in the presence of a metal ion, preferably Zn ion, or a compound releasing a metal ion, preferably Zn ion.

16. The molecule according to any one of claims 8 to 15, wherein said molecule is administered in the presence of a ZnSO₄.

17. The molecule according to any one of claims 8 to 16, wherein said molecule is administered to a human or an animal body, in particular a mammal.

18. A pharmaceutical composition comprising one or more molecules as defined in any one of claims 8 to 17, for use in a treatment of intellectual disability in Down syndrome (DS).

19. A pharmaceutical composition comprising one or more molecules as defined in any one of claims 8 to 18, said composition optionally further comprising a pharmaceutically active molecule limiting intellectual disability in DS, and for example a molecule inhibiting *Dyrk1a* gene and/or DYRK1A protein.


SEQUENCE LISTING

<110> Centre national de la recherche scientifique
      Université de Strasbourg
      INSTITUT NATIONAL DE LA SANTE ET DE LA RECHERCHE MEDICALE
      (INSERM)
      UNIVERSITE DE BRETAGNE OCCIDENTALE

<120> Treatment of Down syndrome

<130> BET 18P0176

<160> 23

<170> BiSSAP 1.3.6

<210> 1
<211> 551
<212> PRT
<213> Homo sapiens

<220>
<223> CBS_HUMAN Cystathionine beta-synthase

<400> 1

<210> 2
<211> 2037
<212> DNA
<213> Saccharomyces cerevisiae


<220>
<223> gene for cystathionine beta-synthase

<400> 2
aagcttcagt tgcattctaa ccttatcaca acaacttcaa cttcacccaa gtaaggataa      60

tcagctctgt cgtgactgat aaatgctata tccggcatat gcagtccaca cggcattacc     120

gtttcactaa tttattgcca tcttcctcca cagttttgca ccgaaagaaa aaaagaaacc     180

aacaccgaaa atttttttct cctaaaggtt aaagtaaacg caaggcactt caccaggctt     240

gtatatataa tgtcgtgatg cttctatgcc aaagtaaaag gcaacacttg aagatttcgt     300

tgtaggccac ttgctcaaag gacatctaga taaatacgac gtaagaataa aaatgactaa     360

atctgagcag caagccgatt caagacataa cgttatcgac ttagttggta acaccccatt     420

gatcgcactg aaaaaattgc ctaaggcttt gggtatcaaa ccacaaattt atgctaagct     480

ggaactatac aatccaggtg gttccatcaa agacagaatt gccaagtcta tggtggaaga     540

agctgaagct tccggtagaa ttcatccttc cagatctact ctgatcgaac ctacttctgg     600

taacaccggt atcggtctag ctttaatcgg cgccatcaaa ggttacagaa ctatcatcac 660

cttgccggaa aaaatgtcta acgagaaagt ttctgtccta aaggctctgg gtgctgaaat 720

catcagaact ccaactgctg ctgcctggga ttctccagaa tcacatattg gtgttgctaa 780

gaagttggaa aaagagattc ctggtgctgt tatacttgac caatataaca atatgatgaa 840

cccagaagct cattactttg gtactggtcg cgaaatccaa agacagctag aagacttgaa 900

tttatttgat aatctacgcg ctgttgttgc tggtgctggt actggtggga ctattagcgg 960

tatttccaag tacttgaaag aacagaatga taagatccaa atcgttggtg ctgacccatt 1020

cggttcaatt ttagcccaac ctgaaaactt gaataagact gatatcactg actacaaagt 1080

tgagggtatt ggttatgatt ttgttcctca ggttttggac agaaaattaa ttgatgtttg 1140

gtataagaca gacgacaagc cttctttcaa atacgccaga caattgattt ctaacgaagg 1200

tgtcttggtg ggtggttctt ccggttctgc cttcactgcg gttgtgaaat actgtgaaga 1260

ccaccctgaa ctgactgaag atgatgtcat tgttgccata ttcccagatt ccatcaggtc 1320

gtacctaacc aaattcgtcg atgacgaatg gttgaaaaag aacaatttgt gggatgatga 1380

cgtgttggcc cgttttgact cttcaaagct ggaggcttcg acgacaaaat acgctgatgt 1440

gtttggtaac gctactgtaa aggatcttca cttgaaaccg gttgtttccg ttaaggaaac 1500

cgctaaggtc actgatgtta tcaagatatt aaaagacaat ggctttgacc aattgcctgt 1560

gttgactgaa gacggcaagt tgtctggttt agttactctc tctgagcttc taagaaaact 1620

atcaatcaat aattcaaaca acgacaacac tataaagggt aaatacttgg acttcaagaa 1680

attaaacaat ttcaatgatg tttcctctta caacgaaaat aaatccggta agaagaagtt 1740

tattaaattc gatgaaaact caaagctatc tgacttgaat cgtttctttg aaaaaaactc 1800

atctgccgtt atcactgatg gcttgaaacc aatccatatc gttactaaga tggatttact 1860

gagctactta gcataaataa gaacccacgc ttcaaataaa agcaaacata gaagcaaaat 1920

ccgtcattcc tttcctattc aattgcaccg ttctctttat ataactactt aattaaatag 1980

cgcctatacg aagcagcatt gttctattat ttttacaaat tccttatcat cggatcc 2037

<210> 3
<211> 32
<212> DNA
<213> Unknown

<220>
<223> primer Bam-Cys4-F

<400> 3
cgggatcccg atgactaaat ctgagcagca ag 32

<210> 4
<211> 35
<212> DNA
<213> Unknown

<220>
<223> Primer Xho-Cys4-R

<400> 4
gcctcgagtc ttatgctaag tagctcagta aatcc                                35

<210> 5
<211> 20
<212> DNA
<213> Unknown

<220>
<223> Primer Cre-fo

<400> 5
ctgcattacc ggtcgatgca                                                 20

<210> 6
<211> 20
<212> DNA
<213> Unknown

<220>
<223> Primer Cre-re

<400> 6
acgttcaccg gcatcaacgt                                                 20

<210> 7
<211> 22
<212> DNA
<213> Unknown

<220>
<223> Primer Myo-fo

<400> 7
atcctggaga tggaccactt gg                                              22

<210> 8
<211> 31
<212> DNA
<213> Unknown

<220>
<223> Primer Myo-re

<400> 8
gatgagtttg gacaaaccac aactagaatg c                                    31

<210> 9
<211> 20
<212> DNA
<213> Unknown

<220>
<223> Primer hCBS-fo

<400> 9
ctcccgggac gaattcgcac                                                    20

<210> 10
<211> 28
<212> DNA
<213> Unknown

<220>
<223> Primer hCBS-re

<400> 10
tcttgttgat tctgaccata ggggtgtc                                           28

<210> 11
<211> 23
<212> DNA
<213> Unknown

<220>
<223> Primer pA-fo (c)

<400> 11
atcctggaga tggaccactt cgc                                                23

<210> 12
<211> 31
<212> DNA
<213> Unknown

<220>
<223> Primer pA-re

<400> 12
gatgagtttg gacaaaccac aactagaatg c                                       31

<210> 13
<211> 20
<212> DNA
<213> Unknown

<220>
<223> Primer Tg(Prp-Gfp-CBS)-fo

<400> 13
gcattctgcc ttcctagtgg                                                    20

<210> 14
<211> 20
<212> DNA
<213> Unknown

<220>
<223> Primer Tg(Prp-Gfp-CBS)-re

<400> 14
gctccttggc ttccttatcc                                                    20

<210> 15
<211> 22
<212> DNA
<213> Unknown

<220>
<223> Primer Cbs-commun-fo

<400> 15
ggtctggaat tcactatgta gc                                                 22

<210> 16
<211> 21
<212> DNA
<213> Unknown

<220>
<223> Primer Cbs-wt-re

<400> 16
aagagcccag cagaatgaac a                                                  21

<210> 17
<211> 19
<212> DNA
<213> Unknown

<220>
<223> Primer Cbs-tm1Unc-re

<400> 17
gaggtcgacg gtatcgata                                                     19

<210> 18
<211> 24
<212> DNA
<213> Unknown

<220>

<223> Primer Tg(CBS)-fo

<400> 18
tgtttcacca aggaaatatt gaga                                              24


<210> 19
<211> 20
<212> DNA
<213> Unknown


<220>
<223> Primer Tg(CBS)-re

<400> 19
aaatgccgct gattgttcac                                                   20


<210> 20
<211> 22
<212> DNA
<213> Unknown


<220>
<223> Primer Tg(Dyrk1a)-fo

<400> 20
tgggccaagc agttaggagt tt                                                22


<210> 21
<211> 25
<212> DNA
<213> Unknown


<220>
<223> Primer Tg(Dyrk1a)-re

<400> 21
ccatgattac gccaagctat ttagg                                             25


<210> 22
<211> 22
<212> DNA
<213> Unknown


<220>
<223> Primer mFez-fo

<400> 22
gcttcgggag caggtaccct at                                                22


<210> 23
<211> 22
<212> DNA
<213> Unknown

<220>
<223> Primer MFez-re

<400> 23
caagatctga ggctcgccaa gt                                                22